# EUROPEAN PATENT APPLICATION

(11) **EP 3 586 865 A1**
(43) Date of publication of application: **01.01.2020**
(21) Application number: 18179178.1
(22) Date of filing: 21.06.2018
(51) Int. Cl.: A61K 38/17, A61K 39/395

(54) **COMPLEMENT ANAPHYLATOXIN BINDERS AND THEIR USE IN TREATMENT OF A SUBJECT HAVING AN OCULAR WOUND AND/OR FIBROSIS**

(71) Applicant: Charité - Universitätsmedizin Berlin, 10117 Berlin (DE)
(72) Inventor: Brockmann, Tobias, 10623 Berlin (DE); Bertelmann, Eckart, 10407 Berlin (DE); Pleyer, Uwe, 13465 Berlin (DE)
(74) Representative: Kilger, Ute

(57) **Abstract**

Subject matter of the present invention is a binder, e.g. protein or protein fragment, binding to complement-anaphylatoxin C5a and/or C3a and/or C4a and thereby inhibiting the activity of C5a and/or C3a and/or C4a for use in the treatment of a subject having an ocular wound and/or fibrosis.

SEQUENCE LISTING

<110>  Charité - Universitätsmedizin Berlin

<120>  Complement anaphylatoxin binders and their use in treatment of a
       subject having an occular wound and/or fibrosis

<130>  C75928EP

<160>  50

<170>  PatentIn version 3.5

<210>  1
<211>  337
<212>  PRT
<213>  Homo sapiens

<400>  1

<210>  2
<211>  350
<212>  PRT
<213>  Homo sapiens

<400>  2

Met Asn Ser Phe Asn Tyr Thr Thr Pro Asp Tyr Gly His Tyr Asp Asp
1               5                   10                  15

Lys Asp Thr Leu Asp Leu Asn Thr Pro Val Asp Lys Thr Ser Asn Thr
            20                  25                  30

Leu Arg Val Pro Asp Ile Leu Ala Leu Val Ile Phe Ala Val Val Phe
        35                  40                  45

Leu Val Gly Val Leu Gly Asn Ala Leu Val Val Trp Val Thr Ala Phe
    50                  55                  60

Glu Ala Lys Arg Thr Ile Asn Ala Ile Trp Phe Leu Asn Leu Ala Val
65                  70                  75                  80

Ala Asp Phe Leu Ser Cys Leu Ala Leu Pro Ile Leu Phe Thr Ser Ile
                85                  90                  95

Val Gln His His His Trp Pro Phe Gly Gly Ala Ala Cys Ser Ile Leu
            100                 105                 110

Pro Ser Leu Ile Leu Leu Asn Met Tyr Ala Ser Ile Leu Leu Leu Ala
        115                 120                 125

Thr Ile Ser Ala Asp Arg Phe Leu Leu Val Phe Lys Pro Ile Trp Cys
    130                 135                 140

Gln Asn Phe Arg Gly Ala Gly Leu Ala Trp Ile Ala Cys Ala Val Ala
145                 150                 155                 160

Trp Gly Leu Ala Leu Leu Leu Thr Ile Pro Ser Phe Leu Tyr Arg Val
                165                 170                 175

Val Arg Glu Glu Tyr Phe Pro Pro Lys Val Leu Cys Gly Val Asp Tyr
            180                 185                 190

Ser His Asp Lys Arg Arg Glu Arg Ala Val Ala Ile Val Arg Leu Val
        195                 200                 205

Leu Gly Phe Leu Trp Pro Leu Leu Thr Leu Thr Ile Cys Tyr Thr Phe
    210                 215                 220

Ile Leu Leu Arg Thr Trp Ser Arg Arg Ala Thr Arg Ser Thr Lys Thr
225                 230                 235                 240

Leu Lys Val Val Val Ala Val Val Ala Ser Phe Phe Ile Phe Trp Leu
                245                 250                 255

Pro Tyr Gln Val Thr Gly Ile Met Met Ser Phe Leu Glu Pro Ser Ser
            260                 265                 270

Pro Thr Phe Leu Leu Leu Asn Lys Leu Asp Ser Leu Cys Val Ser Phe
        275                 280                 285

Ala Tyr Ile Asn Cys Cys Ile Asn Pro Ile Ile Tyr Val Val Ala Gly
    290                 295                 300

Gln Gly Phe Gln Gly Arg Leu Arg Lys Ser Leu Pro Ser Leu Leu Arg
305                 310                 315                 320

Asn Val Leu Thr Glu Glu Ser Val Val Arg Glu Ser Lys Ser Phe Thr
                325                 330                 335

Arg Ser Thr Val Asp Thr Met Ala Gln Lys Thr Gln Ala Val
            340                 345                 350

<210>  3
<211>  482
<212>  PRT
<213>  Homo sapiens

<400>  3
   Met Ala Ser Phe Ser Ala Glu Thr Asn Ser Thr Asp Leu Leu Ser Gln
   1               5                   10                  15
   Pro Trp Asn Glu Pro Pro Val Ile Leu Ser Met Val Ile Leu Ser Leu
               20                  25                  30
   Thr Phe Leu Leu Gly Leu Pro Gly Asn Gly Leu Val Leu Trp Val Ala
           35                  40                  45
   Gly Leu Lys Met Gln Arg Thr Val Asn Thr Ile Trp Phe Leu His Leu
       50                  55                  60
   Thr Leu Ala Asp Leu Leu Cys Cys Leu Ser Leu Pro Phe Ser Leu Ala
   65                  70                  75                  80
   His Leu Ala Leu Gln Gly Gln Trp Pro Tyr Gly Arg Phe Leu Cys Lys
                   85                  90                  95
   Leu Ile Pro Ser Ile Ile Val Leu Asn Met Phe Ala Ser Val Phe Leu
               100                 105                 110
   Leu Thr Ala Ile Ser Leu Asp Arg Cys Leu Val Val Phe Lys Pro Ile
           115                 120                 125
   Trp Cys Gln Asn His Arg Asn Val Gly Met Ala Cys Ser Ile Cys Gly
       130                 135                 140
   Cys Ile Trp Val Val Ala Phe Val Met Cys Ile Pro Val Phe Val Tyr
   145                 150                 155                 160
   Arg Glu Ile Phe Thr Thr Asp Asn His Asn Arg Cys Gly Tyr Lys Phe
                   165                 170                 175
   Gly Leu Ser Ser Ser Leu Asp Tyr Pro Asp Phe Tyr Gly Asp Pro Leu
   180 185 190
   Glu Asn Arg Ser Leu Glu Asn Ile Val Gln Pro Pro Gly Glu Met Asn
   195 200 205
   Asp Arg Leu Asp Pro Ser Ser Phe Gln Thr Asn Asp His Pro Trp Thr
   210 215 220
   Val Pro Thr Val Phe Gln Pro Gln Thr Phe Gln Arg Pro Ser Ala Asp
   225 230 235 240
   Ser Leu Pro Arg Gly Ser Ala Arg Leu Thr Ser Gln Asn Leu Tyr Ser
   245 250 255
   Asn Val Phe Lys Pro Ala Asp Val Val Ser Pro Lys Ile Pro Ser Gly
   260 265 270
   Phe Pro Ile Glu Asp His Glu Thr Ser Pro Leu Asp Asn Ser Asp Ala
   275 280 285
   Phe Leu Ser Thr His Leu Lys Leu Phe Pro Ser Ala Ser Ser Asn Ser
   290 295 300
   Phe Tyr Glu Ser Glu Leu Pro Gln Gly Phe Gln Asp Tyr Tyr Asn Leu
   305 310 315 320
   Gly Gln Phe Thr Asp Asp Asp Gln Val Pro Thr Pro Leu Val Ala Ile
   325 330 335
   Thr Ile Thr Arg Leu Val Val Gly Phe Leu Leu Pro Ser Val Ile Met
   340 345 350
   Ile Ala Cys Tyr Ser Phe Ile Val Phe Arg Met Gln Arg Gly Arg Phe
   355 360 365
   Ala Lys Ser Gln Ser Lys Thr Phe Arg Val Ala Val Val Val Val Ala
   370 375 380
   Val Phe Leu Val Cys Trp Thr Pro Tyr His Ile Phe Gly Val Leu Ser
   385 390 395 400
   Leu Leu Thr Asp Pro Glu Thr Pro Leu Gly Lys Thr Leu Met Ser Trp
   405 410 415
   Asp His Val Cys Ile Ala Leu Ala Ser Ala Asn Ser Cys Phe Asn Pro
   420                 425                 430
   Phe Leu Tyr Ala Leu Leu Gly Lys Asp Phe Arg Lys Lys Ala Arg Gln
           435                 440                 445
   Ser Ile Gln Gly Ile Leu Glu Ala Ala Phe Ser Glu Glu Leu Thr Arg
       450                 455                 460
   Ser Thr His Cys Pro Ser Asn Asn Val Ile Ser Glu Arg Asn Ser Thr
   465                 470                 475                 480
   Thr Val

<210>  4
<211>  344
<212>  PRT
<213>  Mus musculus

<400>  4

Met Met Asn His Thr Thr Ser Glu Tyr Tyr Asp Tyr Glu Tyr Asp His
1               5                   10                  15

Glu His Tyr Ser Asp Leu Pro Asp Val Pro Val Asp Cys Pro Ala Gly
            20                  25                  30

Thr Cys Phe Thr Ser Asp Val Tyr Leu Ile Val Leu Leu Val Leu Tyr
        35                  40                  45

Ala Ala Val Phe Leu Val Gly Val Pro Gly Asn Thr Leu Val Ala Trp
    50                  55                  60

Val Thr Trp Lys Glu Ser Arg His Arg Leu Gly Ala Ser Trp Phe Leu
65                  70                  75                  80

His Leu Thr Met Ala Asp Leu Leu Cys Cys Val Ser Leu Pro Phe Leu
                85                  90                  95

Ala Val Pro Ile Ala Gln Lys Gly His Trp Pro Tyr Gly Ala Ala Gly
            100                 105                 110

Cys Trp Leu Leu Ser Ser Ile Thr Ile Leu Ser Met Tyr Ala Ser Val
        115                 120                 125

Leu Leu Leu Thr Gly Leu Ser Gly Asp Leu Phe Leu Leu Ala Phe Arg
    130                 135                 140

Pro Ser Trp Lys Gly Ala Asp His Arg Thr Phe Gly Val Arg Val Val

145                 150                 155                 160

Gln Ala Ser Ser Trp Met Leu Gly Leu Leu Leu Thr Val Pro Ser Ala
                165                 170                 175

Val Tyr Arg Arg Leu Leu Gln Glu His Tyr Pro Pro Arg Leu Val Cys
            180                 185                 190

Gly Ile Asp Tyr Gly Gly Ser Val Ser Ala Glu Val Ala Ile Thr Thr
        195                 200                 205

Val Arg Phe Leu Phe Gly Phe Leu Gly Pro Leu Val Phe Met Ala Gly
    210                 215                 220

Cys His Gly Ile Leu Gln Arg Gln Met Ala Arg Arg His Trp Pro Leu
225                 230                 235                 240

Gly Thr Ala Val Val Val Gly Phe Phe Ile Cys Trp Thr Pro Tyr His
                245                 250                 255

Val Leu Arg Val Ile Ile Ala Ala Ala Pro Pro His Ser Leu Leu Leu
            260                 265                 270

Ala Arg Val Leu Glu Ala Glu Pro Leu Phe Asn Gly Leu Ala Leu Ala
        275                 280                 285

His Ser Ala Leu Asn Pro Ile Met Phe Leu Tyr Phe Gly Arg Lys Gln
    290                 295                 300

Leu Cys Lys Ser Leu Gln Ala Ala Cys His Trp Ala Leu Arg Asp Pro
305                 310                 315                 320

Gln Asp Glu Glu Ser Ala Val Thr Lys Val Ser Ile Ser Thr Ser His
                325                 330                 335

Glu Met Val Ser Glu Met Pro Val
            340

<210>  5
<211>  350
<212>  PRT
<213>  Mus musculus

<400>  5

Met Asp Pro Ile Asp Asn Ser Ser Phe Glu Ile Asn Tyr Asp His Tyr
1               5                   10                  15

Gly Thr Met Asp Pro Asn Ile Pro Ala Asp Gly Ile His Leu Pro Lys

20                  25                  30

Arg Gln Pro Gly Asp Val Ala Ala Leu Ile Ile Tyr Ser Val Val Phe
        35                  40                  45

Leu Val Gly Val Pro Gly Asn Ala Leu Val Val Trp Val Thr Ala Phe
    50                  55                  60

Glu Ala Arg Arg Ala Val Asn Ala Ile Trp Phe Leu Asn Leu Ala Val
65                  70                  75                  80

Ala Asp Leu Leu Ser Cys Leu Ala Leu Pro Val Leu Phe Thr Thr Val
                85                  90                  95

Leu Asn His Asn Tyr Trp Tyr Phe Asp Ala Thr Ala Cys Ile Val Leu
            100                 105                 110

Pro Ser Leu Ile Leu Leu Asn Met Tyr Ala Ser Ile Leu Leu Leu Ala
        115                 120                 125

Thr Ile Ser Ala Asp Arg Phe Leu Leu Val Phe Lys Pro Ile Trp Cys
    130                 135                 140

Gln Lys Val Arg Gly Thr Gly Leu Ala Trp Met Ala Cys Gly Val Ala
145                 150                 155                 160

Trp Val Leu Ala Leu Leu Leu Thr Ile Pro Ser Phe Val Tyr Arg Glu
                165                 170                 175

Ala Tyr Lys Asp Phe Tyr Ser Glu His Thr Val Cys Gly Ile Asn Tyr
            180                 185                 190

Gly Gly Gly Ser Phe Pro Lys Glu Lys Ala Val Ala Ile Leu Arg Leu
        195                 200                 205

Met Val Gly Phe Val Leu Pro Leu Leu Thr Leu Asn Ile Cys Tyr Thr
    210                 215                 220

Phe Leu Leu Leu Arg Thr Trp Ser Arg Lys Ala Thr Arg Ser Thr Lys
225                 230                 235                 240

Thr Leu Lys Val Val Met Ala Val Val Ile Cys Phe Phe Ile Phe Trp
                245                 250                 255

Leu Pro Tyr Gln Val Thr Gly Val Met Ile Ala Trp Leu Pro Pro Ser
            260                 265                 270

Ser Pro Thr Leu Lys Arg Val Glu Lys Leu Asn Ser Leu Cys Val Ser
        275                 280                 285

Leu Ala Tyr Ile Asn Cys Cys Val Asn Pro Ile Ile Tyr Val Met Ala
    290                 295                 300

Gly Gln Gly Phe His Gly Arg Leu Leu Arg Ser Leu Pro Ser Ile Ile
305                 310                 315                 320

Arg Asn Ala Leu Ser Glu Asp Ser Val Gly Arg Asp Ser Lys Thr Phe
                325                 330                 335

Thr Pro Ser Thr Thr Asp Thr Ser Thr Arg Lys Ser Gln Ala
            340                 345                 350

<210>  6
<211>  477
<212>  PRT
<213>  Mus musculus

<400>  6

Met Glu Ser Phe Asp Ala Asp Thr Asn Ser Thr Asp Leu His Ser Arg
1               5                   10                  15

Pro Leu Phe Gln Pro Gln Asp Ile Ala Ser Met Val Ile Leu Gly Leu
            20                  25                  30

Thr Cys Leu Leu Gly Leu Leu Gly Asn Gly Leu Val Leu Trp Val Ala
        35                  40                  45

Gly Val Lys Met Lys Thr Thr Val Asn Thr Val Trp Phe Leu His Leu
    50                  55                  60

Thr Leu Ala Asp Phe Leu Cys Cys Leu Ser Leu Pro Phe Ser Leu Ala
65                  70                  75                  80

His Leu Ile Leu Gln Gly His Trp Pro Tyr Gly Leu Phe Leu Cys Lys
                85                  90                  95

Leu Ile Pro Ser Ile Ile Ile Leu Asn Met Phe Ala Ser Val Phe Leu
            100                 105                 110

Leu Thr Ala Ile Ser Leu Asp Arg Cys Leu Ile Val His Lys Pro Ile
        115                 120                 125

Trp Cys Gln Asn His Arg Asn Val Arg Thr Ala Phe Ala Ile Cys Gly
    130                 135                 140

Cys Val Trp Val Val Ala Phe Val Met Cys Val Pro Val Phe Val Tyr
145                 150                 155                 160

Arg Asp Leu Phe Ile Met Asp Asn Arg Ser Ile Cys Arg Tyr Asn Phe
                165                 170                 175

Asp Ser Ser Arg Ser Tyr Asp Tyr Trp Asp Tyr Val Tyr Lys Leu Ser
            180                 185                 190

Leu Pro Glu Ser Asn Ser Thr Asp Asn Ser Thr Ala Gln Leu Thr Gly
        195                 200                 205

His Met Asn Asp Arg Ser Ala Pro Ser Ser Val Gln Ala Arg Asp Tyr
    210                 215                 220

Phe Trp Thr Val Thr Thr Ala Leu Gln Ser Gln Pro Phe Leu Thr Ser
225                 230                 235                 240

Pro Glu Asp Ser Phe Ser Leu Asp Ser Ala Asn Gln Gln Pro His Tyr
                245                 250                 255

Gly Gly Lys Pro Pro Asn Val Leu Thr Ala Ala Val Pro Ser Gly Phe
            260                 265                 270

Pro Val Glu Asp Arg Lys Ser Asn Thr Leu Asn Ala Asp Ala Phe Leu
        275                 280                 285

Ser Ala His Thr Glu Leu Phe Pro Thr Ala Ser Ser Gly His Leu Tyr
    290                 295                 300

Pro Tyr Asp Phe Gln Gly Asp Tyr Val Asp Gln Phe Thr Tyr Asp Asn
305                 310                 315                 320

His Val Pro Thr Pro Leu Met Ala Ile Thr Ile Thr Arg Leu Val Val
                325                 330                 335

Gly Phe Leu Val Pro Phe Phe Ile Met Val Ile Cys Tyr Ser Leu Ile
            340                 345                 350

Val Phe Arg Met Arg Lys Thr Asn Phe Thr Lys Ser Arg Asn Lys Thr
        355                 360                 365

Phe Arg Val Ala Val Ala Val Val Thr Val Phe Phe Ile Cys Trp Thr
    370                 375                 380

Pro Tyr His Leu Val Gly Val Leu Leu Leu Ile Thr Asp Pro Glu Ser
385                 390                 395                 400

Ser Leu Gly Glu Ala Val Met Ser Trp Asp His Met Ser Ile Ala Leu
                405                 410                 415

Ala Ser Ala Asn Ser Cys Phe Asn Pro Phe Leu Tyr Ala Leu Leu Gly
            420                 425                 430

Lys Asp Phe Arg Lys Lys Ala Arg Gln Ser Ile Lys Gly Ile Leu Glu
        435                 440                 445

Ala Ala Phe Ser Glu Glu Leu Thr His Ser Thr Asn Cys Thr Gln Asp
    450                 455                 460

Lys Ala Ser Ser Lys Arg Asn Asn Met Ser Thr Asp Val
465                 470                 475

<210>  7
<211>  14
<212>  PRT
<213>  Artificial Sequence

<220>
<223>  Conserved Sequence Fragment characteristic for human and mouse
       C5L2, C5AR1 and C3AR

<400>  7

Phe Leu Val Gly Val Pro Gly Asn Ala Met Val Ala Trp Val
1               5                   10

<210>  8
<211>  10
<212>  PRT
<213>  Artificial Sequence

<220>
<223>  Conserved Sequence Fragment characteristic for human and mouse
       C5L2, C5AR1 and C3AR

<400>  8

Ala Asp Leu Leu Cys Cys Leu Ser Leu Pro
1               5                   10

<210>  9
<211>  9
<212>  PRT
<213>  Artificial Sequence

<220>
<223>  Conserved Sequence Fragment characteristic for human and mouse
       C5L2, C5AR1 and C3AR

<400>  9

Met Tyr Ala Ser Val Leu Leu Leu Ala

1               5


<210>  10
<211>  9
<212>  PRT
<213>  Artificial Sequence

<220>
<223>  Conserved Sequence Fragment characteristic for human and mouse
       C5L2, C5AR1 and C3AR

<400>  10

Leu Ala Leu Leu Leu Thr Val Pro Ser
1               5


<210>  11
<211>  8
<212>  PRT
<213>  Artificial Sequence

<220>
<223>  Conserved Sequence Fragment characteristic for human and mouse
       C5L2, C5AR1 and C3AR

<400>  11

Phe Phe Val Cys Trp Ala Pro Tyr
1               5


<210>  12
<211>  6
<212>  PRT
<213>  Artificial Sequence

<220>
<223>  Conserved Sequence Fragment characteristic for human and mouse
       C5L2, C5AR1 and C3AR

<400>  12

Gly His Trp Pro Tyr Gly
1               5


<210>  13
<211>  11
<212>  PRT
<213>  Artificial Sequence

<220>
<223>  Conserved Sequence Fragment characteristic for human and mouse
       C5L2, C5AR1 and C3AR

<400>  13

Tyr Ser Asp Leu Ser Asp Arg Pro Val Asp Cys
1               5                   10

<210>  14
<211>  11
<212>  PRT
<213>  Artificial Sequence

<220>
<223>  Conserved Sequence Fragment characteristic for human and mouse
       C5L2, C5AR1 and C3AR

<400>  14

Tyr Ser Asp Leu Pro Asp Val Pro Val Asp Cys
1               5                   10


<210>  15
<211>  9
<212>  PRT
<213>  Artificial Sequence

<220>
<223>  Conserved Sequence Fragment characteristic for human and mouse
       C5L2, C5AR1 and C3AR

<400>  15

Thr Leu Asp Leu Asn Thr Pro Val Asp
1               5


<210>  16
<211>  9
<212>  PRT
<213>  Artificial Sequence

<220>
<223>  Conserved Sequence Fragment characteristic for human and mouse
       C5L2, C5AR1 and C3AR

<400>  16

Thr Met Asp Pro Asn Ile Pro Ala Asp
1               5


<210>  17
<211>  10
<212>  PRT
<213>  Artificial Sequence

<220>
<223>  Conserved Sequence Fragment characteristic for human and mouse
       C5L2, C5AR1 and C3AR

<400>  17

Pro Leu Val Ala Ile Thr Ile Thr Arg Leu
1               5                   10


<210>  18
<211>  23
<212>  PRT

<213>  Artificial Sequence

<220>
<223>  N-terminal fragment of human C5L2

<400>  18

Met Gly Asn Asp Ser Val Ser Tyr Glu Tyr Gly Asp Tyr Ser Asp Leu
1               5                   10                  15

Ser Asp Arg Pro Val Asp Cys
            20

<210>  19
<211>  29
<212>  PRT
<213>  Artificial Sequence - Sequence 19

<220>
<223>  N-terminal fragment of mouse C5L2

<400>  19

Met Met Asn His Thr Thr Ser Glu Tyr Tyr Asp Tyr Glu Tyr Asp His
1               5                   10                  15

Glu His Tyr Ser Asp Leu Pro Asp Val Pro Val Asp Cys
            20                  25

<210>  20
<211>  74
<212>  PRT
<213>  Homo sapiens

<400>  20

Thr Leu Gln Lys Lys Ile Glu Glu Ile Ala Ala Lys Tyr Lys His Ser
1               5                   10                  15

Val Val Lys Lys Cys Cys Tyr Asp Gly Ala Cys Val Asn Asn Asp Glu
            20                  25                  30

Thr Cys Glu Gln Arg Ala Ala Arg Ile Ser Leu Gly Pro Arg Cys Ile
        35                  40                  45

Lys Ala Phe Thr Glu Cys Cys Val Val Ala Ser Gln Leu Arg Ala Asn
    50                  55                  60

Ile Ser His Lys Asp Met Gln Leu Gly Arg
65                  70

<210>  21
<211>  73
<212>  PRT

<213>  Homo sapiens

<400>  21

Thr Leu Gln Lys Lys Ile Glu Glu Ile Ala Ala Lys Tyr Lys His Ser
1               5                   10                  15

Val Val Lys Lys Cys Cys Tyr Asp Gly Ala Cys Val Asn Asn Asp Glu
            20                  25                  30

Thr Cys Glu Gln Arg Ala Ala Arg Ile Ser Leu Gly Pro Arg Cys Ile
        35                  40                  45

Lys Ala Phe Thr Glu Cys Cys Val Val Ala Ser Gln Leu Arg Ala Asn
    50                  55                  60

Ile Ser His Lys Asp Met Gln Leu Gly
65                  70

<210>  22
<211>  14
<212>  PRT
<213>  Artificial Sequence - Sequence 22

<220>
<223>  Artificial Sequence - Sequence 22

<400>  22

Thr Leu Gln Lys Lys Ile Glu Glu Ile Ala Ala Lys Tyr Lys
1               5                   10

<210>  23
<211>  13
<212>  PRT
<213>  Artificial Sequence

<220>
<223>  Artificial Sequence - Prior Art

<400>  23

His Ser Val Val Lys Lys Cys Cys Tyr Asp Gly Ala Cys
1               5                   10

<210>  24
<211>  5
<212>  PRT
<213>  Artificial Sequence

<220>
<223>  Artificial Sequence - Prior Art

<400>  24

Val Asn Asn Asp Glu

1               5


<210>  25
<211>  8
<212>  PRT
<213>  Artificial Sequence

<220>
<223>  Artificial Sequence - Prior Art

<400>  25

Thr Cys Glu Gln Arg Ala Ala Arg
1               5


<210>  26
<211>  4
<212>  PRT
<213>  Artificial Sequence

<220>
<223>  Artificial Sequence

<400>  26

Ile Ser Leu Gly
1


<210>  27
<211>  22
<212>  PRT
<213>  Artificial Sequence

<220>
<223>  Artificial Sequence - Prior Art

<400>  27

Pro Arg Cys Ile Lys Ala Phe Thr Glu Cys Cys Val Val Ala Ser Gln
1               5                   10                  15


Leu Arg Ala Asn Ile Ser
            20


<210>  28
<211>  7
<212>  PRT
<213>  Artificial Sequence

<220>
<223>  Artificial Sequence - Prior Art

<400>  28

His Lys Asp Met Gln Leu Gly
1               5

<210>  29
<211>  8
<212>  PRT
<213>  Artificial Sequence

<220>
<223>  Artificial Sequence - Prior Art

<400>  29

His Lys Asp Met Gln Leu Gly Arg
1               5


<210>  30
<211>  14
<212>  PRT
<213>  Artificial Sequence

<220>
<223>  Artificial Sequence - Prior Art

<400>  30

Cys Cys Tyr Asp Gly Ala Cys Val Asn Asn Asp Glu Thr Cys
1               5                   10


<210>  31
<211>  33
<212>  PRT
<213>  Artificial Sequence

<220>
<223>  Artificial Sequence - Prior Art

<400>  31

Cys Tyr Asp Gly Ala Cys Val Asn Asn Asp Glu Thr Cys Glu Gln Arg
1               5                   10                  15


Ala Ala Arg Ile Ser Leu Gly Pro Arg Cys Ile Lys Ala Phe Thr Glu
            20                  25                  30


Cys



<210>  32
<211>  22
<212>  PRT
<213>  Artificial Sequence

<220>
<223>  Artificial Sequence - Prior Art

<400>  32

Cys Glu Gln Arg Ala Ala Arg Ile Ser Leu Gly Pro Arg Cys Ile Lys
1               5                   10                  15

Ala Phe Thr Glu Cys Cys
            20

<210>  33
<211>  18
<212>  PRT
<213>  Artificial Sequence

<220>
<223>  Artificial Sequence - Prior Art

<400>  33

Tyr Asp Gly Ala Cys Val Asn Asn Asp Glu Thr Cys Glu Gln Arg Ala
1               5                   10                  15

Ala Arg

<210>  34
<211>  18
<212>  PRT
<213>  Artificial Sequence

<220>
<223>  Artificial Sequence - Prior Art

<400>  34

Cys Tyr Asp Gly Ala Cys Val Asn Asn Asp Glu Thr Cys Glu Gln Arg
1               5                   10                  15

Ala Ala

<210>  35
<211>  9
<212>  PRT
<213>  Artificial Sequence

<220>
<223>  Artificial Sequence - Prior art

<220>
<221>  X
<222>  (1)..(1)
<223>  X is selected from thegroup consisting of N, H, D, F, K, Y and T

<220>
<221>  X
<222>  (2)..(2)
<223>  X is selected from thegroup consisting of D, L, Y and H

<220>
<221>  X
<222>  (7)..(7)

<223>  X is selected from thegroup consisting of Q, E, K

<220>
<221>  X
<222>  (9)..(9)
<223>  X is selected from thegroup consisting of A, V, and L

<400>  35

Xaa Xaa Glu Thr Cys Glu Xaa Arg Xaa
1               5


<210>  36
<211>  7
<212>  PRT
<213>  Artificial Sequence

<220>
<223>  Artificial Sequence - prior art


<220>
<221>  X
<222>  (1)..(1)
<223>  X is either S, H, P, or N

<220>
<221>  X
<222>  (2)..(2)
<223>  X is either H or N

<220>
<221>  X
<222>  (4)..(4)
<223>  X is either D, N, H, P, or G

<220>
<221>  X
<222>  (5)..(5)
<223>  X is either M, L, I, or V

<220>
<221>  X
<222>  (6)..(6)
<223>  X is either Q, L, or I

<400>  36

Xaa Xaa Lys Xaa Xaa Xaa Leu
1               5


<210>  37
<211>  7
<212>  PRT
<213>  Artificial Sequence

<220>
<223>  Artificial Sequence - Prior Art


<220>

<221>  X
<222>  (1)..(6)
<223>  Residues 1, 2, 4, 5, 6 are selected from the same groups as in
       SEQ ID No. 36

<400>  37

Xaa Xaa Lys Xaa Xaa Xaa Ile
1               5


<210>  38
<211>  9
<212>  PRT
<213>  Artificial Sequence

<220>
<223>  Artificial Sequence - Prior Art

<400>  38

Asn Asp Glu Thr Cys Glu Gln Arg Ala
1               5


<210>  39
<211>  7
<212>  PRT
<213>  Artificial Sequence

<220>
<223>  Artificial Sequence - Prior Art

<400>  39

Ser His Lys Asp Met Gln Leu
1               5


<210>  40
<211>  7
<212>  PRT
<213>  Artificial Sequence

<220>
<223>  Artificial Sequence - Prior Art

<400>  40

Asp Glu Thr Cys Glu Gln Arg
1               5


<210>  41
<211>  40
<212>  DNA
<213>  Artificial Sequence

<220>
<223>  Artificial Sequence from US 20120065254

<400>  41
gcgauguggu ggugaagggu uguugggugu cgacgcacgc                              40

<210>  42
<211>  7
<212>  PRT
<213>  Artificial Sequence

<220>
<223>  C5a amino acid including sequence from Yatime, L. et al., Nat
       Commun. 2015

<400>  42

Lys Lys Cys Cys Tyr Asp Gly
1               5


<210>  43
<211>  77
<212>  PRT
<213>  Homo sapiens

<400>  43

Ser Val Gln Leu Thr Glu Lys Arg Met Asp Lys Val Gly Lys Tyr Pro
1               5                   10                  15


Lys Glu Leu Arg Lys Cys Cys Glu Asp Gly Met Arg Glu Asn Pro Met
            20                  25                  30


Arg Phe Ser Cys Gln Arg Arg Thr Arg Phe Ile Ser Leu Gly Glu Ala
        35                  40                  45


Cys Lys Lys Val Phe Leu Asp Cys Cys Asn Tyr Ile Thr Glu Leu Arg
    50                  55                  60


Arg Gln His Ala Arg Ala Ser His Leu Gly Leu Ala Arg
65                  70                  75


<210>  44
<211>  8
<212>  PRT
<213>  Artificial Sequence

<220>
<223>  Artificial Sequence

<400>  44

Ala Ser His Leu Gly Leu Ala Arg
1               5


<210>  45
<211>  9
<212>  PRT
<213>  Artificial Sequence

<220>
<223>  Artificial Sequence

<400>  45

Ala Ser His Leu Gly Leu Ala Arg Gly
1               5


<210>  46
<211>  13
<212>  PRT
<213>  Artificial Sequence

<220>
<223>  Artificial Sequence

<400>  46

Arg Gln His Ala Arg Ala Ser His Leu Gly Leu Ala Arg
1               5                   10


<210>  47
<211>  14
<212>  PRT
<213>  Artificial Sequence

<220>
<223>  Artificial Sequence

<400>  47

Arg Gln His Ala Arg Ala Ser His Leu Gly Leu Ala Arg Gly
1               5                   10


<210>  48
<211>  77
<212>  PRT
<213>  Homo sapiens

<400>  48

Asn Val Asn Phe Gln Lys Ala Ile Asn Glu Lys Leu Gly Gln Tyr Ala
1               5                   10                  15


Ser Pro Thr Ala Lys Arg Cys Cys Gln Asp Gly Val Thr Arg Leu Pro
            20                  25                  30


Met Met Arg Ser Cys Glu Gln Arg Ala Ala Arg Val Gln Gln Pro Asp
        35                  40                  45


Cys Arg Glu Pro Phe Leu Ser Cys Cys Gln Phe Ala Glu Ser Leu Arg
    50                  55                  60


Lys Lys Ser Arg Asp Lys Gly Gln Ala Gly Leu Gln Arg
65                  70                  75

<210>  49
<211>  380
<212>  PRT
<213>  Homo sapiens

<400>  49

Thr Leu Glu Ile Pro Gly Asn Ser Asp Pro Asn Met Ile Pro Asp Gly
1               5                   10                  15

Asp Phe Asn Ser Tyr Val Arg Val Thr Ala Ser Asp Pro Leu Asp Thr
            20                  25                  30

Leu Gly Ser Glu Gly Ala Leu Ser Pro Gly Gly Val Ala Ser Leu Leu
        35                  40                  45

Arg Leu Pro Arg Gly Cys Gly Glu Gln Thr Met Ile Tyr Leu Ala Pro
    50                  55                  60

Thr Leu Ala Ala Ser Arg Tyr Leu Asp Lys Thr Glu Gln Trp Ser Thr
65                  70                  75                  80

Leu Pro Pro Glu Thr Lys Asp His Ala Val Asp Leu Ile Gln Lys Gly
                85                  90                  95

Tyr Met Arg Ile Gln Gln Phe Arg Lys Ala Asp Gly Ser Tyr Ala Ala
            100                 105                 110

Trp Leu Ser Arg Asp Ser Ser Thr Trp Leu Thr Ala Phe Val Leu Lys
        115                 120                 125

Val Leu Ser Leu Ala Gln Glu Gln Val Gly Gly Ser Pro Glu Lys Leu
    130                 135                 140

Gln Glu Thr Ser Asn Trp Leu Leu Ser Gln Gln Gln Ala Asp Gly Ser
145                 150                 155                 160

Phe Gln Asp Pro Cys Pro Val Leu Asp Arg Ser Met Gln Gly Gly Leu
                165                 170                 175

Val Gly Asn Asp Glu Thr Val Ala Leu Thr Ala Phe Val Thr Ile Ala
            180                 185                 190

Leu His His Gly Leu Ala Val Phe Gln Asp Glu Gly Ala Glu Pro Leu
        195                 200                 205

Lys Gln Arg Val Glu Ala Ser Ile Ser Lys Ala Asn Ser Phe Leu Gly
    210                 215                 220

Glu Lys Ala Ser Ala Gly Leu Leu Gly Ala His Ala Ala Ala Ile Thr
225                 230                 235                 240

Ala Tyr Ala Leu Ser Leu Thr Lys Ala Pro Val Asp Leu Leu Gly Val
                245                 250                 255

Ala His Asn Asn Leu Met Ala Met Ala Gln Glu Thr Gly Asp Asn Leu
            260                 265                 270

Tyr Trp Gly Ser Val Thr Gly Ser Gln Ser Asn Ala Val Ser Pro Thr
        275                 280                 285

Pro Ala Pro Arg Asn Pro Ser Asp Pro Met Pro Gln Ala Pro Ala Leu
    290                 295                 300

Trp Ile Glu Thr Thr Ala Tyr Ala Leu Leu His Leu Leu Leu His Glu
305                 310                 315                 320

Gly Lys Ala Glu Met Ala Asp Gln Ala Ser Ala Trp Leu Thr Arg Gln
                325                 330                 335

Gly Ser Phe Gln Gly Gly Phe Arg Ser Thr Gln Asp Thr Val Ile Ala
            340                 345                 350

Leu Asp Ala Leu Ser Ala Tyr Trp Ile Ala Ser His Thr Thr Glu Glu
        355                 360                 365

Arg Gly Leu Asn Val Thr Leu Ser Ser Thr Gly Arg
    370                 375                 380

<210>  50
<211>  6
<212>  PRT
<213>  Artificial Sequence

<220>
<223>  Artificial Sequence 50

<400>  50

Pro Cys Pro Val Leu Asp
1               5

## Description

Subject matter of the present invention is a binder, e.g. a protein or protein fragment or peptide, binding to complement anaphylatoxin C5a and/or C3a and/or C4a and thereby inhibiting the activity of C5a and/or C3a and/or C4a for use in the treatment of a subject having an ocular wound and/ or fibrosis.

### Prior Art

Degenerative eye disorders, which are associated to a severe loss of visual acuity very often are the result of misguided angiogenesis or wound healing/fibrogenesis (Friedlander M. J Clin Invest. 2007). While the treatment of vascular eye disorders was substantially improved by profound research and the introduction of anti-VEGF therapeutics (Vascular Endothelial Growth Factor, VEGF), (Lim LS et al. Lancet 2012; Feigl B. Prog Retin Eye Res 2009; Joussen AM et al. FASEB J 2004) the treatment of fibrotic eye disorders is still lacking on therapeutic approaches.

Misguided wound healing and fibrogenesis is of outmost relevance in particular on the cornea. Corneal fibrosis results in a loss of optical transparency that substantially impedes vision and may result in blindness of the affected eye. Corneal scars can occur on base of a corneal herpetic infection, microbial keratitis, mechanic or chemical affection, stromal keratopathies, persistent corneal edema due to endothelial decompensation or corneal graft failure. Today, in most cases a penetrating corneal transplantation is the only therapeutic option to restore vison. In this regard, the number of performed corneal transplantations and the number of severe corneal complications, associated with corneal fibrosis, due to contact lenses or due to corneal laser refractive surgeries is increasing. Notwithstanding the above, the life-time risk to suffer a relevant ocular trauma, with corneal affection accounts for 20%.(Ljubimov AV et al. Prog Retin Eye Res 2015) Current therapeutic options to inhibit ocular fibrogenesis are very limited and primarily refer to corticosteroids and ciclosporin A (CSA). Both substances possess a non-specific efficacy, which is accompanied with various adverse effects. In this regard, corticosteroids induce cataract development and intraocular pressure elevation but also evoke systemic adverse events, such as the Cushing syndrome and alterations of blood parameters (glucose). CSA has a slow onset of action, which usually responds too slow to prevent fibrosis, therefore CSA is not feasible for an acute treatment, its topical application is accompanied with stinging and redness of the eyes and also evokes systemic adverse events, in particular arterial hypertension.

However, this therapeutic dilemma not only relates to the cornea, as mentioned in the examples above, but also to tissue fibrosis in various conditions of misled wound healing and scarring in eye diseases, involving ocular fibroblast and myofibroblasts, which occur in the conjunctiva, sclera, iris, trabecular meshwork, vitreous, retina, choroid and optic nerve head. Furthermore, fundamental pathophysiologic processes involved in fibrosis and scarring, related to fibroblast activation and/or differentiation, are likewise of relevance for fibrotic diseases of the lung, liver, kidney, pancreas, heart, skin and vascular system. Against this background, the establishment of new therapeutic options for the treatment of ocular fibrosis and superordinate fibrotic conditions is of considerable clinical importance.

The physiologic wound healing intervenes several tissue processes and follows a sequence of cell migration and/or transformation, proliferation and modulation of the extracellular matrix; (Ljubimov AV et al. Prog Retin Eye Res 2015) whereas activated fibroblasts and myofibroblasts are the key mediators.(Gabbiani G., J Pathol 2003) During the regular course of wound healing, reversible protein depositions are accumulated within the extracellular matrix.(Wynn TA et al. Nat Med 2012) Yet, in the context of fibrotic remodeling, which is triggered by a dysregulation of pro- and anti-fibrotic cascades, a permanent myofibroblasts activation emerges that may lead to a constant and irreversible deposition of matrix proteins, such as collagen, fibronectin and proteoglycans. (Medzhitov R. Cell 2010; Wynn TA, J Pathol. 2008).

On the basis of the aforementioned, the inhibition of myofibroblasts and their activation may selectively direct wound-healing processes to regular clearance-mechanisms and thereby prevent tissue fibrosis and scarring. However, regarding the inhibition of ocular myofibroblasts, anatomic particularities of the eye have to be considered. First, the blood-ocular barrier prevents the efficacy of systemically applied inhibitors/modulators, especially those based on proteins/peptides. Second, the direct application (e.g. topical, in the form of eye drops) requires the penetration of the inhibitor/modulator into the tissue that is intended to be treated. Therefore the inhibitors/modulators need to as small as to penetrate into the conjunctiva, sclera, iris, trabecular meshwork, vitreous, retina, choroid, or even the optic nerve head. Proteins with a molecular weight of 28-67 kDa are able to penetrate through the cornea with an intact corneal epithelium into the anterior chamber, while proteins with a molecular weight of 60-90 kDa are able to penetrate through the cornea into the anterior chamber after removal of the corneal epithelium.(Thiel MA et al. Clin Exp Immunol 2002) Conventional therapeutic approaches of specific inhibitors, such as monoclonal antibodies (anti-VEGF antibody, bevacizumab: 149 kDa), do not fulfill these conditions.

It was the objection of the present invention to provide a treatment of a subject having an ocular wound or fibrosis that overcomes the shortcomings of the prior art methods.

Therefore, the aim of the present invention is to provide a substance that inhibits the process of fibroblast/myofibroblast activation and/or transdifferentiation, i.e. at least essentially inhibits the process of fibroblast/myofibroblast activation and/or transdifferentiation and has preferably a molecular weight less than 90 kDa, preferably less than 80 kDa or less, preferably less than 70 kDa or less, more preferably less than 60 kDa or less, more preferably less than 50 kDa or less, more preferably less than 45 kDa or less, more preferably less than 40 kDa or less, even more preferably less than 35 kDa or less, even more preferably less than 30 kDa or less, even more preferably less than 25 kDa or less, even more preferably less than 20 kDa or less, even more preferably less than 15 kDa or less, and even more preferably less than 10 kDa or less.

Subject matter of the present invention is a binder, in particular a protein or protein fragment, binding to complement-anaphylatoxin C5a and/or C3a and/or C4a and preferably thereby inhibiting the activity of C5a and/or C3a and/or C4a for use in the treatment of a subject having an ocular wound or fibrosis.

Inhibiting the activity of C5a and/or C3a and/or C4a means inhibiting essentially the action of C5a and/or C3a and/or C4a by binding to C5a and/or C3a and/or C4a.

Subject matter of the present invention is a binder for use in the treatment of a subject having an ocular wound or fibrosis wherein said binder is administered to promote wound healing, in particular corneal wound healing.

A binder maybe selected from the group comprising a protein or a fragment thereof, a peptide, a non-IgG scaffold in particular an aptamer, oligonucleotides, an antibody or antibody-like proteins, peptidomimetics or a fragment thereof.

Antibodies, antibody-like proteins or binders, as described above, may bind to several overlapping peptide fragments of a complement component C5a protein (e.g., several overlapping fragments of a human C5a protein having the amino acid sequence depicted in SEQ ID No.: 20 or SEQ ID No.: 21. The antibodies, antibody-like proteins or binders may also bind only to a human C5a at an epitope within or overlapping with a fragment of the protein having the amino acid sequence, according to SEQ ID No's.: 22-34 (see e.g., Cooketal. (2010) Acta Cryst D66:190-197 and as descibed in US 2016/0159892). Furthermore, the antibody, antibody-like protein or binder may also bind to an epitope of C5a formed by amino acid sequences according to SEQ ID No's: 35-40 (SEQ ID No.: 35: X₁X₂ETCEX₃RX₄, SEQ ID No.: 36: X₅X₆KX₇X₈X₉L and SEQ ID No.: 37: X₅X₆KX₇X₈X₉I), wherein X₁ is selected from the group consisting of N, H, D, F, K, Y, and T; X₂ is selected from the group consisting of D, L, Y, and H; X₃ is selected from the group consisting of Q, E, and K; X₄ is selected from the group consisting of A, V, and L; X₅ is selected from the group consisting of S, H, P, and N; X₆ is selected from the group consisting of H and N; X₇ is selected from the group consisting of D, N, H, P, and G; X₈ is selected from the group consisting of M, L, I, and V; and X₉ is selected from the group consisting of Q, L, and I (as described in US 2012/0231008, US 2017/0002067, WO 2011/063980 and US8802096).

Antibodies, antibody-like proteins or binders, as described above, may bind to several overlapping peptide fragments of a complement component C3a protein (e.g., several overlapping fragments of a human C3a protein having the amino acid sequence depicted in SEQ ID No.: 43. The antibodies, antibody-like proteins or binders may also bind only to a human C3a at an epitope within or overlapping with a fragment of the protein having the amino acid sequence, according to SEQ ID No's.: 44-47 (see e.g., Hugli TE. J Biol Chem. 1975; Hugli TE et al. PNAS 1977; Payan D et al. J. Exp Med. 1982).

Antibodies, antibody-like proteins or binders, as described above, may bind to several overlapping peptide fragments of a complement component C4a protein (e.g., several overlapping fragments of a human C4a protein having the amino acid sequence depicted in SEQ ID No.: 48 or SEQ ID No.: 49. The antibodies, antibody-like proteins or binders may also bind only to a human C4a at an epitope within or overlapping with a fragment of the protein having the amino acid sequence, according to SEQ ID No.: 50 (see e.g., Yu CY et al. EMBO J. 1986; Nettesheim D.G. et al. PNAS 1988).

A peptide is defined as a compound consisting of at least two amino acids in which the carboxyl group of one acid is linked to the amino group of the other, which can be created by peptide synthesis. Thus, as defined for this invention a peptide may have from 2 to 50 amino acids. A protein comprises more than 50 amino acids, according to the definition of this invention.

A protein is defined as a macromolecule consisting of one or more chains of amino acids, or peptides, linked by peptide bonds, which can be created by protein ligation of two or more peptides, by recombinant expression or by protein biosynthesis.

A protein fragment is defined as a section of an amino acids sequence that derives from a protein that served as template.

An antibody according to the present invention is a protein including one or more polypeptides substantially encoded by immunoglobulin genes that specifically binds an antigen. The recognized immunoglobulin genes include the kappa, lambda, alpha (IgA), gamma (IgG₁, IgG₂, IgG₃, IgG₄), delta (IgD), epsilon (IgE) and mu (IgM) constant region genes, as well as the myriad immunoglobulin variable region genes. Full-length immunoglobulin light chains are generally about 25 kDa or 214 amino acids in length. Full-length immunoglobulin heavy chains are generally about 50 kDa or 446 amino acid in length. Light chains are encoded by a variable region gene at the NH2-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')₂, as well as bifunctional hybrid antibodies and single chains (*e.g.,* Lanzavecchia et al., Eur. J. Immunol. 17:105,1987; Huston et al., Proc. Natl. Acad. Sci. U.S.A., 85:5879-5883, 1988; Bird et al., Science 242:423-426, 1988; Hood et al., Immunology, Benjamin, N.Y., 2nd ed., 1984; Hunkapiller and Hood, Nature 323:15-16,1986). An immunoglobulin light or heavy chain variable region includes a framework region interrupted by three hypervariable regions, also called complementarity determining regions (CDR's) (see, Sequences of Proteins of Immunological Interest, E. Kabat et al., U.S. Department of Health and Human Services, 1983). As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art, *e.g*., see U.S. Patent No. 5,807,715. A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor". In one embodiment, all the CDRs are from the donor immunoglobulin in a humanized immunoglobulin. Constant regions need not be present, but if they are, they must be substantially identical to human immunoglobulin constant regions, *i.e.,* at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions, which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering (*e.g.,* see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, *e.g.,* Dower et al., PCT Publication No. WO91/17271; McCafferty et al., PCT Publication No. WO92/001047; and Winter, PCT Publication No. WO92/20791), or selected from a human combinatorial monoclonal antibody library (see the Morphosys website). Human antibodies can also be prepared by using transgenic animals carrying a human immunoglobulin gene (for example, see Lonberg et al., PCT Publication No. WO93/12227; and Kucherlapati, PCT Publication No. WO91/10741).

Thus, the antibody according to the present invention may have the formats known in the art. Examples are human antibodies, monoclonal antibodies, humanized antibodies, chimeric antibodies, CDR-grafted antibodies. In a preferred embodiment antibodies according to the present invention are recombinantly produced antibodies as *e.g.* IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains,*e.g.* Fab-dHLX-FSx2; F(ab')2-fragments, scFv-fragments, multimerized multivalent or/and multispecific scFv-fragments, bivalent and/or bispecific diabodies, BITE® (bispecific T-cell engager), trifunctional antibodies, polyvalent antibodies, *e.g.* from a different class than G; single-domain antibodies, *e.g.* nanobodies derived from camelid or fish immunoglobulines and numerous others.

In addition to antibodies other biopolymer scaffolds are well known in the art to complex a target molecule and have been used for the generation of highly target specific biopolymers. Examples are aptamers, spiegelmers, anticalins and conotoxins.

In a preferred embodiment the antibody format is selected from the group comprising Fv fragment, scFv fragment, Fab fragment, scFab fragment, (Fab)2 fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One particular formats is the scFab format.

Non-Ig scaffolds may be protein scaffolds and may be used as antibody mimics as they are capable to bind to ligands or antigenes. Non-Ig scaffolds may be selected from the group comprising tetranectin-based non-Ig scaffolds (*e.g.* described in US 2010/0028995), fibronectin scaffolds (*e.g.* described in EP 1266 025; lipocalin-based scaffolds ((*e.g.* described in WO 2011/154420); ubiquitin scaffolds (*e.g.* described in WO 2011/073214), transferring scaffolds (*e.g.* described in US 2004/0023334), protein A scaffolds (*e.g.* described in EP 2231860), ankyrin repeat based scaffolds (*e.g.* described in WO 2010/060748), microproteins, preferably microproteins forming a cystine knot) scaffolds *(e.g.* described in EP 2314308), Fyn SH3 domain based scaffolds (*e.g.* described in WO 2011/023685) EGFR-A-domain based scaffolds (*e.g.* described in WO 2005/040229) and Kunitz domain based scaffolds (*e.g.* described in EP 1941867).

Non-immunoglobulin (Non-IgG) scaffolds are defined as small antibody alternatives. An aptamer is defined as a molecule that binds to a specific target and may consist of RNA and/or DNA and/or amino acids (peptide).

An aptamer, may relate to a nucleic acid molecule consisting of RNA and/or DNA, such as disclosed in SEQ ID No.: 41 (5'-GCGAU G(dU)GGU GGU(dG)(dA) AGGGU UGUUG GG(dU)G(dU) CGACG CA(dC)GC-3') and as described in US 2012/0065254, capable of binding to C5a, whereas the binding site of C5a is comprising a C5a amino acid sequence including SEQ ID No.: 42 (see Yatime L. et al. Nat Commun. 2015).

In one embodiment of the invention antibodies according to the present invention may be produced as follows:
A Balb/c mouse was immunized with antigen-100µg Peptide-BSA-Conjugate (BSA = bovine serum albumin) at day 0 and 14 (emulsified in 100µl complete Freund's adjuvant) and 50µg at day 21 and 28 (in 100µl incomplete Freund's adjuvant). Three days before the fusion experiment was performed, the animal received 50µg of the conjugate dissolved in 100µl saline, given as one intraperitoneal and one intravenous injection.

Spenocytes from the immunized mouse and cells of the myeloma cell line SP2/0 were fused with 1 ml 50% polyethylene glycol for 30s at 37°C. After washing, the cells were seeded in 96-well cell culture plates. Hybrid clones were selected by growing in HAT medium (RPMI (Roswell Park Memorial Institute) 1640 culture medium supplemented with 20% fetal calf serum and HAT-Supplement). After two weeks the HAT medium is replaced with HAT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting, the selected cultures were cloned and recloned using the limiting-dilution technique and the isotypes were determined (see also Lane, R.D. (1985). A short-duration polyethylene glycol fusion technique for increasing production of monoclonal antibody-secreting hybridomas. J. Immunol. Meth. 81: 223-228; Ziegler, B. et al.(1996) Glutamate decarboxylase (GAD) is not detectable on the surface of rat islet cells examined by cytofluorometry and complement-dependent antibody-mediated cytotoxicity of monoclonal GAD antibodies, Horm. Metab. Res. 28: 11-15).

Antibodies may be produced by means of phage display according to the following procedure:
The human naive antibody gene libraries HAL7/8 were used for the isolation of recombinant single chain F-Variable domains (scFv) against peptide. The antibody gene libraries were screened with a panning strategy comprising the use of peptides containing a biotin tag linked via two different spacers to the peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatant from the cultivation of these clonal strains has been directly used for an antigen ELISA testing (see Hust, M., Meyer, T., Voedisch, B., Rülker, T., Thie, H., El-Ghezal, A., Kirsch, M.I., Schütte, M., Helmsing, S., Meier, D., Schirrmann, T., Dübel, S., 2011. A human scFv antibody generation pipeline for proteome research. Journal of Biotechnology 152, 159-170; Schütte, M., Thullier, P., Pelat, T., Wezler, X., Rosenstock, P., Hinz, D., Kirsch, M.I.,Hasenberg, M., Frank, R., Schirrmann, T., Gunzer, M., Hust, M., Dübel, S., 2009. Identification of a putative Crf splice variant and generation of recombinant antibodies for the specific detection of Aspergillus fumigatus. PLoS One 4, e6625).

Humanization of murine antibodies may be conducted according to the following procedure:

For humanization of an antibody of murine origin the antibody sequence is analyzed for the structural interaction of framework regions (FR) with the complementary determining regions (CDR) and the antigen. Based on structural modeling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modeling (see Almagro JC, Fransson J., 2008. Humanization of antibodies. Front Biosci. 2008 Jan 1;13:1619-33).

In a preferred embodiment the antibody format is selected from the group comprising Fv fragment, scFv fragment, Fab fragment, scFab fragment, F(ab)₂ fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is scFab format.

In one embodiment of the invention said binder, e.g. a protein or protein fragment thereof, according to the present invention binds to C5a and C3a and thereby inhibiting the activity of C5a and C3a

In one embodiment of the invention said binder, e.g. a protein or protein fragment according to the present invention binds to C5a and C4a and thereby inhibiting the activity of, C5a and C4a.

In one embodiment of the invention said binder, e.g. a protein or protein fragment according to the present invention binds to C3a and C4a and thereby inhibiting the activity of C3a and C4a.

In one embodiment of the invention said binder, e.g. a protein or protein fragment according to the present invention binds to C5a and C3a and C4a and thereby inhibiting the activity of C5a and C3a and C4a.

In one specific embodiment of the invention said binder, e.g. a protein or protein fragment is a soluble complement receptor protein or protein fragment. In one specific embodiment of the invention said protein or protein fragment/peptide is a recombinant soluble complement receptor protein or synthetic protein fragment/peptide.

A soluble receptor is defined as the extracellular portion of the receptor, (Fischer DG. Science 1993) in case of C3a it is the extracellular portion of the C3a anaphylatoxin chemotactic receptor (C3aR1), in case of C5a it is the extracellular portion of the C5a anaphylatoxin chemotactic receptor 1 and/or 2 (C5aR1/CD88 and C5aR2/C5L2). A separate specific C4a receptor is not known, therefore in case of C4a it is the extracellular portion of the C3a anaphylatoxin chemotactic receptor (C3aR1) and/or the C5a anaphylatoxin chemotactic receptor 1 and/or 2 (C5aR1/CD88 and/or C5aR2/C5L2).

In one embodiment of the invention said binder, e.g. protein or protein fragment/peptide, according to the present invention binds specifically to complement-anaphylatoxin C5a and/or C3a and/or C4a,

The term "specific binding" is defined as a protein-ligand binding affinity with a dissociation constant of 1 mM or less, preferably 100 µM or less, preferably 50 µM or less, preferably 30 µM or less, preferably 20 µM or less, preferably 10 µM or less, preferably 5 µM or less, more preferably 1 µM or less, more preferably 900 nM or less, more preferably 800 nM or less, more preferably 700 nM or less, more preferably 600 nM or less, more preferably 500 nM or less, more preferably 400 nM or less, more preferably 300 nM or less, more preferably 200 nM or less, even more preferably 100 nM or less, even more preferably 90 nM or less, even more preferably 80 nM or less, even more preferably 70 nM or less, even more preferably 60 nM or less, even more preferably 50 nM or less, even more preferably 40 nM or less, even more preferably 30 nM or less, even more preferably 20 nM or less, and even more preferably 10 nM or less; determined by a radioligand binding assay (Cain SA, Monk PN, J Biol Chem. 2002) or surface plasmon resonance (BIAcore) (Colley CS et al. MAbs. 2018; as described in US 2012/0065254) or ELISA-based binding assay (Michelfelder S., J Am Soc Nephrol. 2018).

The term "inhibiting the activity", with regard to a protein or protein fragment/peptide, a non-IgG scaffold, an aptamer, oligonucleotides, an antibody or antibody-like proteins, peptidomimetics, or a fragment thereof according to the present invention, refers to the characteristic of inhibiting the process of fibroblast/myofibroblast activation and/or transdifferentiation in the presence of C5a and/or C3a and/or C4a stimulation. For this purpose, fibroblasts (e.g. human corneal keratocytes) incubated for 24 hours with C3a and/or C4a and/or C5a at a concentration of 0.1 µg/ml in DMEM (Dulbecco's Modified Eagle Medium) growth medium without fetal bovine serum ('stimulation control') are being compared to fibroblasts, which are incubated under the same conditions but with the addition of a protein or protein fragment/peptide, a non-IgG scaffold, an aptamer, oligonucleotides, an antibody or antibody-like proteins, peptidomimetics, or a fragment thereof according to the present invention that shall be tested for its efficacy ('inhibition control'). After stimulation, the proportion (given in percentages) of myofibroblasts in a monolayered fibroblast cell culture is being determined by alpha smooth muscle actin (aSMA) immunocytochemistry staining, using anti-aSMA antibodies. Hereby myofibroblasts become apparent as cells that stain positive for aSMA in the cytoplasma. A protein or protein fragment/peptide, a non-IgG scaffold, an aptamer, an antibody or a fragment thereof, according to the present invention, is defined as effective, considering its optimal conditions and concentration, by the means of "inhibiting the activity" of myofibroblast activation if the proportion of myofibroblasts in the 'inhibition control' can be reduced preferably by at least 10%, more preferably by at least 20%, even more preferably by at least 25%, even more preferably by at least 30%, even more preferably by at least 35%, even more preferably by at least 40%, even more preferably by at least 45%, even more preferably by at least 50%, even more preferably by at least 55%, even more preferably by at least 60%, and even more preferably by at least 65%, compared to the proportion of myofibroblasts in the 'stimulation control'.

In one embodiment of the invention said binder is a protein or protein fragment is selected from the group comprising human C5L2 protein according to SEQ ID No.: 1, a protein or fragment that is at least 60% identical to the full-length amino acid sequence of human C5L2 protein of SEQ ID No.:1, human C5aR1 protein according to SEQ ID No.: 2, a protein or fragment that is at least 60% identical to the full-length amino acid sequence of human C5aR1 protein of SEQ ID No.: 2, human C3aR protein according to SEQ ID No.: 3, a protein or fragment that is at least 60% identical to the full-length amino acid sequence of human C3aR protein of SEQ ID No.: 3, mouse C5L2 protein according to SEQ ID No.: 4, a protein or fragment that is at least 60% identical to the full-length amino acid sequence of mouse C5L2 protein of SEQ ID No.:4, mouse C5aR1 protein according to SEQ ID No.: 5, a protein or fragment that is at least 60% identical to the full-length amino acid sequence of mouse C5aR1 protein of SEQ ID No.: 5, mouse C3aR protein according to SEQ ID No.: 6, and a protein or fragment that is at least 60% identical to the full-length amino acid sequence of mouse C3aR protein of SEQ ID No.: 6.

In a specific embodiment of the invention the identity to the respective full-length amino acid sequence is least 65%, or at least 70%, or at least 75 %, or at least 80 %, or at least 85 %, or at least 90 %, or at least 95 %, or at least 97 %, or at least 98 %, or at least 99 %.

The extent of a identity between two amino acid sequences is defined as the result of heuristic algorithms, such as FASTA (Lipman DJ et al. Science 1985, Pearson WR et al. PNAS 1988) and basic local alignment search tool (BLAST).(Lobo I. Nature Education 2008). The identity of a protein/peptide or protein fragment that shall be tested, to an amino acid sequence according to SEQ ID No's.: 1-17, is 100% if the protein/peptide or protein fragment that is tested is identical (respectively has a BLAST result of 100% identity) or contains a fragment identical (respectively has a BLAST result of 100% identity) to SEQ ID No's.: 1-17.

In one embodiment of the invention said protein or protein fragment comprises at least one conserved region selected from the group comprising an amino acid sequence according to SEQ ID No.:7, an amino acid sequence according to SEQ ID No.:8, an amino acid sequence according to SEQ ID No.:9, an amino acid sequence according to SEQ ID No.:10, an amino acid sequence according to SEQ ID No.: 11, an amino acid sequence according to SEQ ID No.:12, an amino acid sequence according to SEQ ID No.:13, an amino acid sequence according to SEQ ID No.:14, an amino acid sequence according to SEQ ID No.:15, an amino acid sequence according to SEQ ID No.:16, an amino acid sequence according to SEQ ID No.:17, and a protein or fragment that is at least 60% identical to any of the amino acid sequences according to SEQ ID No's.:7-17.

In one embodiment of the invention said protein or protein fragment comprises at least two conserved regions selected from the group comprising an amino acid sequence according to SEQ ID No.:7, an amino acid sequence according to SEQ ID No.:8, an amino acid sequence according to SEQ ID No.:9, an amino acid sequence according to SEQ ID No.:10, an amino acid sequence according to SEQ ID No.:11, an amino acid sequence according to SEQ ID No.:12, an amino acid sequence according to SEQ ID No.:13, an amino acid sequence according to SEQ ID No.:14, an amino acid sequence according to SEQ ID No.:15, an amino acid sequence according to SEQ ID No.:16, an amino acid sequence according to SEQ ID No.:17, and a protein or fragment that is at least 60% identical to any of the amino acid sequences according to SEQ IS No's.:7-17.

In another embodiment of the invention said protein or protein fragment comprises at least three of the before-mentioned conserved regions, or at least four of the before- mentioned conserved regions, or at least five of the before-mentioned conserved regions, or six of the before- mentioned conserved regions.

In one embodiment of the invention the conserved regions exhibit at least at least 65%, or at least 75 %, or at least 80%, or at least 85%, or at least 90 %, or at least 95 %, or at least 97 %, or at least 98 % , or at least 99 % sequence identity to any of the before-mentioned amino acids according to SEQ ID No.: 7-17.

**Table 1 provides an overview of sequence identities, determined by BLAST, between corresponding amino acid sequences of conserved sequence fragments (SEQ ID No's.: 7-17) characteristic for human and mouse C5L2, C5AR1 and C3AR.**

| **Table 1.** Identities of corresponding amino acid sequences corresponding. | | | | | | | |
|---|---|---|---|---|---|---|---|
| **SEQ ID** | **Sequence** | **human C5L2** | **mouse C5L2** | **human C5AR1** | **mouse C5AR1** | **human C3AR** | **mouse C3AR** |
| No.: 7 | FLVGVPGNAMVAWV | **100%** | **86%** | **79%** | **86%** | **64%** | **50%** |
| No.: 8 | ADLLCCLSLP | **100%** | **90%** | **70%** | **80%** | **100%** | **90%** |
| No.: 9 | MYASVLLLA | **100%** | **89%** | **89%** | **89%** | **67%** | **67%** |
| No.: 10 | LALLLTVPS | **100%** | **89%** | **89%** | **89%** | 22% | 33% |
| No.: 11 | FFVCWAPY | **100%** | **75%** | **63%** | **63%** | **75%** | **75%** |
| No.: 12 | GHWPYG | **100%** | **100%** | **67%** | 17% | **83%** | **100%** |
| No.: 13 | YSDLSDRPVDC | **100%** | **82%** | 45% | 18% | 0% | 36% |
| No.: 14 | YSDLPDVPVDC | **82%** | **100%** | 45% | 36% | 0% | 27% |
| No.: 15 | TLDLNTPVD | 33% | 56% | **100%** | 56% | 0% | 33% |
| No.: 16 | TMDPNIPAD | 22% | 44% | 56% | **100%** | 0% | 11% |
| No.: 17 | PLVAITITRL | 30% | 0% | 0% | 40% | **100%** | **90%** |
| Bold = Identity to the corresponding amino acid sequences is >60%. | | | | | | | |

Subject matter of the present invention is a composition comprising at least one binder, e.g. a proteins or protein fragment, according to the present invention for use in the treatment of a subject having an ocular wound and/or fibrosis.

Subject matter of the present invention is a composition comprising at least two binders, e.g. two proteins/peptides or protein fragments, according to the present invention for use in the treatment of a subject having an ocular wound and/or fibrosis.

Subject matter of the present invention is a composition comprising at least three binders, e.g. proteins/peptides or protein fragments, according to the present inventions for use in the treatment of a subject having an ocular wound and/or fibrosis.

One binder, e.g. protein or protein fragment, may contain one or multiple binding sites for C5a and/or C3a and/or C4a. According to Table 1, the number of binding sites may vary, depending on the number of comprised sequences selected from SEQ ID No.: 1-17.

In this regard, a composition of more than one binder, e.g. protein/peptide or protein fragment comprising SED ID No.: 1-17 expands the inhibiting effect on C3a-, C4a- and C5a-dependent activities. In particular, the combination of proteins or protein fragments deriving from primarily C3a-binding moieties, such as SEQ ID No's: 8, 12 and 17, with proteins or protein fragments deriving from primarily C5a-binding moieties, such as SEQ ID No's: 7, 9, 10, 11, 13, 14, 15 and 16, are of particular importance.

Subject matter of the present invention is a pharmaceutical composition comprising a binder, e.g. protein or protein fragment, according to the present invention or a composition according to the present invention for use in the treatment of a subject having an ocular wound and/or fibrosis.

The binders of the present invention may be pegylated, or altered in a comparable way, to modify the biological stability and/or half-life of the binder. PEGylation is the process of both covalent and noncovalent attachment or amalgamation of polyethylene glycol (PEG, in pharmacy called macrogol) polymer chains to molecules and macrostructures, such as a drug, therapeutic protein or vesicle, which is then described as PEGylated (pegylated). PEGylation is routinely achieved by the incubation of a reactive derivative of PEG with the target molecule. The covalent attachment of PEG to a drug or therapeutic protein can "mask" the agent from the host's immune system (reducing immunogenicity and antigenicity), and increase its hydrodynamic size (size in solution), which prolongs its circulatory time by reducing renal clearance.

The binders of the present invention may undergo posttranslational or post-synthesis modifications that may comprise i.a. the attachment of sugars, fatty acids, phosphate groups (phosphoryl group, phosphorylation), hydroxyl groups, methyl groups (methylation of proteins), ubiquitin (ubiquitination of proteins), to alter the actual structure of the binder and may enhance its function or stability. These modification may be made on both, the amino (amino terminus) and carboxyl end (carboxyl terminus) of a binder, as well as amino acid side chains (amino acids) within the protein and may be reversible and/or irreversible.

Subject matter are furthermore prodrugs of the binder according to the present invention. A prodrug is a medication or compound that, after administration, is metabolized (i.e., converted within the body) into a pharmacologically active drug. Inactive prodrugs are pharmacologically inactive medications that are metabolized into an active form within the body. Instead of administering a drug directly, a corresponding prodrug might be used instead to improve how a medicine is absorbed, distributed, metabolized, and excreted.

In one embodiment of the invention said pharmaceutical composition is for topical application, i.e. is topically administered.

In one embodiment of the invention said pharmaceutical composition is for intraocular application, i.e. is intraocular administered.

In one embodiment of the invention said pharmaceutical composition is for intravitreal application, i.e. is intravitreal administered.

In one embodiment of the invention said pharmaceutical composition is for subconjunctival application, i.e. is subconjunctival administered.

In one embodiment of the invention said pharmaceutical composition is for intravascular/intravenous application, i.e. is intravascular/intravenous administered.

One embodiment of the present invention is a binder, e.g. protein or protein fragment, according to the present invention or a composition according to the present invention or a pharmaceutical composition according to the present invention for use in the treatment of a subject wherein said subject suffers from a disease selected from the group comprising: conjunctivitis and conjunctival scars (including ocular pemphigoid), scleritis and episcleritis, corneal scars and opacities due to corneal ulcer, keratoconjunctivitis, keratitis, bullous keratopathy, corneal degenerations, iridocyclitis and adhesions of iris and ciliary body, chorioretinal scars/fibrosis due to chorioretinal inflammation or degeneration or haemorrhage or rupture or neovascularization, fibrotic vitreoretinopathies, such as in proliferative vitreoretinopathy, retinopathy of prematurity and diabetic retinopathy; choroidal neovascularization and degenerations of the macula, secondary glaucoma, endophthalmitis, and impairments of wound healing and fibrosis after ocular surgery or trauma, including intraocular foreign bodies.

One embodiment of the present invention is a binder, e.g. protein/peptide or protein fragment, according to the present invention or a composition according to the present invention or a pharmaceutical composition according to the present invention for use in the treatment of a subject wherein said subject suffers from corneal fibrosis.

One embodiment of the present invention is a binder, e.g. protein/peptide or protein fragment, according to the present invention or a composition according to the present invention or a pharmaceutical composition according to the present invention for use in the treatment of a subject wherein said subject suffers from chorioretinal fibrosis.

One embodiment of the present invention is a binder, e.g. protein/peptide or protein fragment, according to the present invention or a composition according to the present invention or a pharmaceutical composition according to the present invention for use in the treatment of a subject wherein said subject suffers from impairments of wound healing and fibrosis after ocular surgery or trauma

One embodiment of the present invention is binder, e.g. protein or protein fragment, according to the present invention or a composition according to the present invention or a pharmaceutical composition according to the present invention for use in the treatment of a subject wherein said subject suffers from a disease selected from the group comprising: (idiopathic) pulmonary fibrosis, dermal keloid formation, sclerodermia, myelofibrosis, kidney-, pancreas- and heart-fibrosis, and fibrosis in (non)-alcoholic steatohepatosis, glomerulonephritis and (ANCA-associated) vasculitis.

One embodiment of the present invention is a binder, e.g. protein or protein fragment, according to the present invention or a composition according to the present invention or a pharmaceutical composition according to the present invention for use in the treatment of a subject wherein said subject suffers from pulmonary fibrosis.

One embodiment of the present invention is a binder, e.g. protein or protein fragment, according to the present invention or a composition according to the present invention or a pharmaceutical composition according to the present invention for use in the treatment of a subject wherein said subject suffers from fibrosis due to glomerulonephritis and/or renal fibrosis

One embodiment of the present invention is a binder, e.g. protein or protein fragment, according to the present invention or a composition according to the present invention or a pharmaceutical composition according to the present invention for use in the treatment of a subject wherein said subject suffers from steatohepatosis and/or liver fibrosis.

The following embodiments are subject of the invention:
1. Binder binding to complement-anaphylatoxin C5a and/or C3a and/or C4a and thereby preferably inhibiting the activity of C5a and/or C3a and/or C4a for use in the treatment of a subject having an ocular wound and/or fibrosis.
2. Binder for use in the treatment of a subject having an ocular wound and/or fibrosis according to embodiment 1 wherein said binder is selected from the group comprising a protein or a fragment thereof, a peptide, a non-IgG scaffold, an aptamer, oligonucleotides, an antibody or antibody-like proteins, peptidomimetics or a fragment thereof.
3. Binder for use in the treatment of a subject having an ocular wound and/or fibrosis according to embodiment 1 or 2 wherein said binder is a protein or a fragment thereof.
4. Binder according to any of embodiments 1 to 3 for use in the treatment of a subject having an ocular wound and/or fibrosis wherein said binder is administered to promote wound healing, in particular corneal wound healing.
5. Binder according to any of embodiments 1 to 4 for use in the treatment of a subject having an ocular wound and/or fibrosis wherein said binder binds to C5a and C3a and thereby essentially inhibiting the activity of C5a and C3a.
6. Binder according to any of embodiments 1 to 5 for use in the treatment of a subject having an ocular wound and/or fibrosis wherein said binder binds to C5a and C4a and thereby essentially inhibiting the activity of C5a and C4a.
7. Binder according to any of embodiments 1 to 6 for use in the treatment of a subject having an ocular wound and/or fibrosis wherein said binder binds to C3a and C4a and thereby essentially inhibiting the activity of C3a and C4a.
8. Binder according to any of embodiments 1 to 7 for use in the treatment of a subject having an ocular wound and/or fibrosis wherein said binder binds to C5a and C3a and C4a and thereby inhibiting the activity of C5a and C3a and C4a.
9. Binder according to any of embodiments 1 to 8 for use in the treatment of a subject having an ocular wound and/or fibrosis wherein said binder is a protein or protein fragment is selected from the group comprising human C5L2 protein according to SEQ ID No.: 1, a protein/peptide or fragment that is at least 60% identical to the full-length amino acid sequence of human C5L2 protein of SEQ ID No.:1, human C5aRl protein according to SEQ ID No.: 2, a protein or fragment that is at least 60% identical to the full-length amino acid sequence of human C5aR1 protein of SEQ ID No.: 2, human C3aR protein according to SEQ ID No.: 3, a protein or fragment that is at least 60% identical to the full-length amino acid sequence of human C3aR protein as of SEQ ID No.: 3, a mouse C5L2 protein according to SEQ ID No.: 4, a protein or fragment that is at least 60% identical to the full-length amino acid sequence of mouse C5L2 protein of SEQ ID No.:4, mouse C5aR1 protein according to SEQ ID No.: 5, a protein or fragment that is at least 60% identical to the full-length amino acid sequence of mouse C5aR1 protein of SEQ ID No.: 5, mouse C3aR protein according to SEQ ID No.: 6, and a protein or fragment that is at least 60% identical to the full-length amino acid sequence of mouse C3aR protein of SEQ ID No.: 6.
10. Binder according to any of embodiments 1 to 9 for use in the treatment of a subject having an ocular wound and/or fibrosis wherein said binder is a protein/peptide or protein fragment and comprises at least one conserved region selected from the group comprising an amino acid sequence according to SEQ ID No.:7, an amino acid sequence according to SEQ ID No.:8, an amino acid sequence according to SEQ ID No.:9, an amino acid sequence according to SEQ ID No.:10, an amino acid sequence according to SEQ ID No.:11, an amino acid sequence according to SEQ ID No.:12, an amino acid sequence according to SEQ ID No.:13, an amino acid sequence according to SEQ ID No.:14, an amino acid sequence according to SEQ ID No.:15, an amino acid sequence according to SEQ ID No.:16, an amino acid sequence according to SEQ ID No.:17, and a protein or fragment that is at least 60% identical to any of the amino acid sequences according to SEQ ID No's.:7-17.
11. Binder according to embodiments 10 for use in the treatment of a subject having an ocular wound and/or fibrosis wherein said binder is a protein/peptide or protein fragment and comprises at least two conserved region selected from the group comprising an amino acid sequence according to SEQ ID No.:7, an amino acid sequence according to SEQ ID No.:8, an amino acid sequence according to SEQ ID No.:9, an amino acid sequence according to SEQ ID No.: 1 0, an amino acid sequence according to SEQ ID No.:11, an amino acid sequence according to SEQ ID No.:12, an amino acid sequence according to SEQ ID No.:13, an amino acid sequence according to SEQ ID No.:14, an amino acid sequence according to SEQ ID No.:15, an amino acid sequence according to SEQ ID No.:16, an amino acid sequence according to SEQ ID No.:17, and a protein or fragment that is at least 60% identical to any of the amino acid sequences according to SEQ ID No's.:7-17.
12. Composition comprising at least two binders, preferably proteins or protein fragments, according to any of embodiments 1 to 11 for use in the treatment of a subject having an ocular wound and/or fibrosis.
13. Composition comprising at least three proteins or protein fragments according to any of embodiments 1 to 9 for use in the treatment of a subject having an ocular wound and/or fibrosis.
14. Pharmaceutical composition comprising a binder according to any of embodiments 1-11 or a composition according to embodiments 12 or 13 for use in the treatment of a subject having an ocular wound and/or fibrosis.
15. Pharmaceutical composition according embodiments 14 wherein said pharmaceutical composition further comprises a carrier and/or an excipient and/or a stabilizer.
16. Pharmaceutical composition according embodiments 14 or 15 for topical application.
17. Pharmaceutical composition according embodiments 14 or 15 for intraocular application.
18. Pharmaceutical composition according embodiments 14 or 15 for intravitrealer application.
19. Pharmaceutical composition according embodiments 14 or 15 for subconjunctivaler application.
20. Binder according to any of embodiments 1-11 or a composition according to embodiments 12 or 13 or a pharmaceutical composition of any of embodiments 14 - 19 for use in the treatment of a subject wherein said subject suffers from a disease selected from the group comprising: conjunctivitis and conjunctival scars (including ocular pemphigoid), scleritis and episcleritis, corneal scars and opacities due to corneal ulcer, keratoconjunctivitis, keratitis, bullous keratopathy, corneal degenerations, iridocyclitis and adhesions of iris and ciliary body, chorioretinal scars/fibrosis due to chorioretinal inflammation or degeneration or haemorrhage or rupture or neovascularization, fibrotic vitreoretinopathies, such as in proliferative vitreoretinopathy, retinopathy of prematurity and diabetic retinopathy; choroidal neovascularization and degenerations of the macula, secondary glaucoma, endophthalmitis, and impairments of wound healing and fibrosis after ocular surgery or trauma, including intraocular foreign bodies.
21. Binder according to any of embodiments 1-11 or a composition according to embodiments 12 or 13 or a pharmaceutical composition of any of embodiments 14 - 19 for use in the treatment of a subject wherein said subject suffers from a disease selected from the group comprising: (idiopathic) pulmonary fibrosis, dermal keloid formation, sclerodermia, myelofibrosis, kidney-, pancreas- and heart-fibrosis, and fibrosis in (non)-alcoholic steatohepatosis, glomerulonephritis and (ANCA-associated) vasculitis.

### FIGURE DESCRIPTION

Figure 1 shows the effect of inhibiting a C3a-mediated myofibroblast activation by human C5L2 protein fragment (hC5L2).
Figure 2 shows the effect of inhibiting inhibition a C5a-mediated myofibroblast activation by human C5L2 protein fragment (hC5L2).
Figure 3 shows the effect of inhibiting a C5a- and C3a-mediated myofibroblast activation by human C5L2 protein fragment (hC5L2).
Figure 4 shows the effect of inhibiting a C3a-mediated myofibroblast activation by mouse C5L2 protein fragment (mC5L2).
Figure 5 shows the effect of inhibiting a C5a-mediated myofibroblast activation by mouse C5L2 protein fragment (mC5L2).
Figure 6 shows the effect of inhibiting a C5a- and C3a-mediated myofibroblast activation by mouse C5L2 protein fragment (mC5L2).
Figure 7 shows the effect of human C5L2 protein fragment concentration on myofibroblasts in the presence of fetal bovine serum (FCS).
Figure 8 shows the effect of mouse C5L2 protein fragment concentration on myofibroblasts in the presence of fetal bovine serum (FCS).
Figure 9 shows the effect of human C5L2 protein fragment concentration on myofibroblasts without fetal bovine serum.
Figure 10 shows the effect of mouse C5L2 protein fragment concentration on myofibroblasts without fetal bovine serum.

### EXAMPLES

### Example 1

### Human C5L2 protein fragment causes inhibition of myofibroblasts activated by C3a

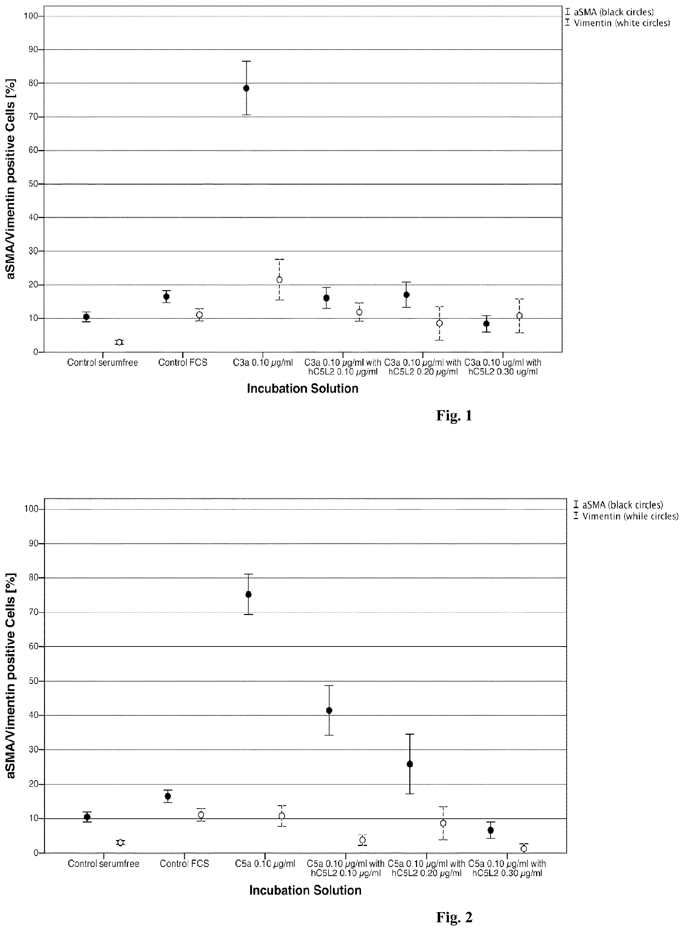

To explore the potential functional role of human C5L2 protein fragment (hC5L2), according to SEQ ID No.: 18, in the treatment of a subject having an ocular wound or fibrosis, the effect of its presence on C3a-activated myofibroblasts was examined. Human corneal keratocytes were stimulated with human C3a for 24 hours and assessed in regard to activated myofibroblasts (Fig. 1). For the detection of activated myofibroblasts aSMA antibodies were used, as well as vimentin antibodies as a marker for extracellular matrix. As a reference group, human corneal keratocytes incubated for 24 hours in DMEM growth medium with and without 10% fetal bovine serum (FCS, fetal calf serum) respectively were used. As shown in Fig. 1, C3a 0.1 µg/ml caused significant activation of myofibroblasts (measured by aSMA positive cells, 74±22%) in comparison with the reference group (serumfree: 10±11%; FCS: 16±14%; p < 0.001 and p < 0.001, respectively). A list of genes, attained from human corneal keratocytes and generated from a gene expression Clariom S human microarray, that have differing expression levels (fold change: ≥2 or ≤ -2) after 24 hours of incubation with human C3a 0.1 µg/ml and DMEM growth medium without fetal bovine serum (serumfree control) is shown in Table 2. Incubation in the presence of human C3a and the human C5L2 protein fragment resulted in significant decrease (hC5L2 0.1 µg/ml: 16±9%; hC5L2 0.2 µg/ml: 17±11%; hC5L2 0.3 µg/ml: 8±7%), compared to C3a-activated myofibroblasts (p < 0.001, p < 0.001 and p < 0.001, respectively). A list of genes, attained from human corneal keratocytes and generated from a gene expression Clariom S human microarray, that have differing expression levels (fold change: ≥2 or ≤ -2) after 24 hours of incubation with human C3a 0.1 µg/ml and human C3a 0.1 µg/ml with human C5L2 protein fragment 0.3 µg/ml, according to SEQ ID No.: 18, is shown in Table 5. Thus, the human C5L2 protein fragment was responsible for causing inhibition of myofibroblasts activated by C3a. Bar = Standard error of the mean.

### Example 2

### Human C5L2 protein fragment causes inhibition of myofibroblasts activated by C5a

To explore the potential functional role of human C5L2 protein fragment (hC5L2), according to SEQ ID No.: 18, in the treatment of a subject having an ocular wound or fibrosis, the effect of its presence on C5a-activated myofibroblasts was examined. Human corneal keratocytes were stimulated with human C5a for 24 hours and assessed in regard to activated myofibroblasts (Fig. 2). For the detection of activated myofibroblasts aSMA antibodies were used, as well as vimentin antibodies as a marker for extracellular matrix. As a reference group, human corneal keratocytes incubated for 24 hours in DMEM growth medium with and without 10% fetal bovine serum (FCS, fetal calf serum) respectively were used. As shown in Fig. 2, C5a 0.1 µg/ml caused significant activation of myofibroblasts (measured by aSMA positive cells, 77±23%) in comparison with the reference group (serumfree: 10±11%; FCS: 16±14%; p < 0.001 and p < 0.001, respectively). A list of genes, attained from human corneal keratocytes and generated from a gene expression Clariom S human microarray, that have differing expression levels (fold change: ≥2 or ≤ -2) after 24 hours of incubation with human C5a 0.1 µg/ml and DMEM growth medium without fetal bovine serum (serumfree control) is shown in Table 3. Incubation in the presence of C5a and the human C5L2 protein fragment resulted in significant decrease of activated myofibroblasts (hC5L2 0.1 µg/ml: 41±22%; hC5L2 0.2 µg/ml: 26±26%; hC5L2 0.3 µg/ml: 7±7%), compared to C5a-activated myofibroblasts (p = 0.001, p < 0.001 and p < 0.001, respectively). A list of genes, attained from human corneal keratocytes and generated from a gene expression Clariom S human microarray, that have differing expression levels (fold change: ≥ or ≤ -2) after 24 hours of incubation with human C5a 0.1 µg/ml and human C5a 0.1 µg/ml with human C5L2 protein fragment 0.3 µg/ml, according to SEQ ID No.: 18, is shown in Table 6. Thus, the human C5L2 protein fragment was responsible for causing inhibition of myofibroblasts activated by C5a. Bar = Standard error of the mean.

### Example 3

### Human C5L2 protein fragment causes inhibition of myofibroblasts activated by C5a and C3a

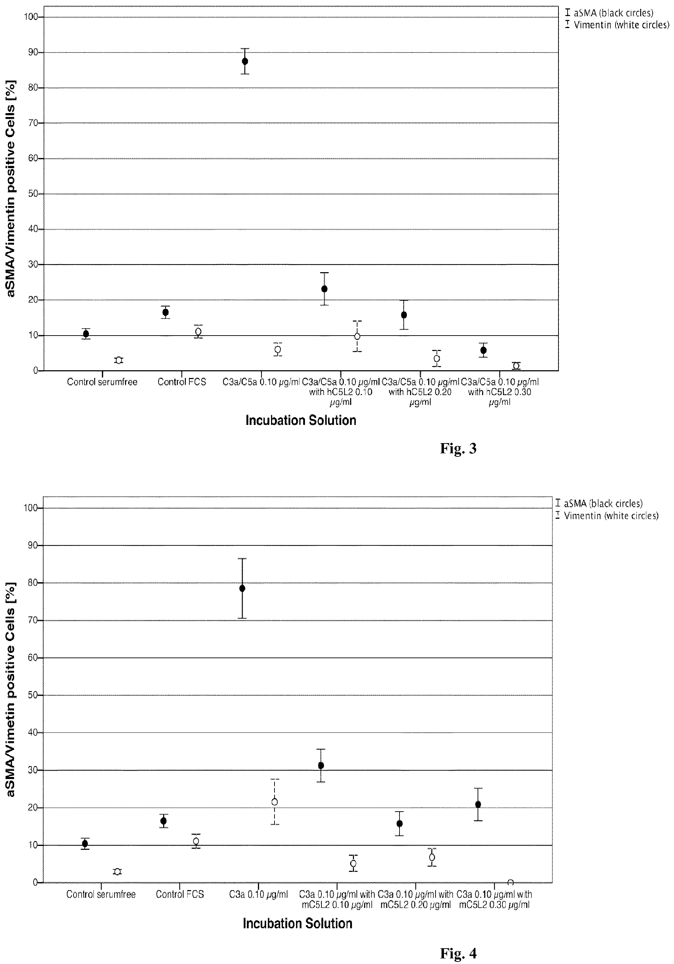

To explore the potential functional role of human C5L2 protein fragment, according to SEQ ID No.: 18, in the treatment of a subject having an ocular wound or fibrosis, the effect of its presence on C5a/C3a-activated myofibroblasts was examined. Human corneal keratocytes were stimulated with human C5a and human C3a respectively for 24 hours and assessed in regard to activated myofibroblasts (Fig. 3). For the detection of activated myofibroblasts aSMA antibodies were used, as well as vimentin antibodies as marker for extracellular matrix. As a reference group, human corneal keratocytes incubated for 24 hours in DMEM growth medium with and without 10% fetal bovine serum (FCS, fetal calf serum) respectively were used. As shown in Fig. 3, C5a and C3a, both at a concentration of 0.1 µg/ml, caused significant activation of myofibroblasts (measured by aSMA positive cells, 87±11%) in comparison with the reference group (serumfree: 10±11%; FCS: 16±14%; p < 0.001 and p < 0.001, respectively). A list of genes, attained from human corneal keratocytes and generated from a gene expression Clariom S human microarray, that have differing expression levels (fold change: ≥2 or ≤ -2) after 24 hours of incubation with human C3a and C5a, both at a concentration of 0.1 µg/ml, and DMEM growth medium without fetal bovine serum (serumfree control) is shown in Table 4. Incubation in the presence of C3a, C5a and the human C5L2 protein fragment resulted in significant decrease of activated myofibroblasts (hC5L2 0.1 µg/ml: 23±14%; hC5L2 0.2 µg/ml: 16±12%; hC5L2 0.3 µg/ml: 6±6%), compared to C5a- and C3a-activated myofibroblasts (p < 0.001, p < 0.001 and p < 0.001, respectively). A list of genes, attained from human corneal keratocytes and generated from a gene expression Clariom S human microarray, that have differing expression levels (fold change: ≥2 or ≤ -2) after 24 hours of incubation with human C3a and C5a, both 0.1 µg/ml, and human C3a and C5a, both 0.1 µg/ml, with human C5L2 protein fragment 0.3 µg/ml, according to SEQ ID No.: 18, is shown in Table 7. Thus, the human C5L2 protein fragment was responsible for causing inhibition of myofibroblasts activated by C5a and C3a. Bar = Standard error of the mean.

### Example 4

### Mouse C5L2 protein fragment causes inhibition of myofibroblasts activated by C3a

To explore the potential functional role of mouse C5L2 protein fragment (mC5L2), according to SEQ ID No.: 19, in the treatment of a subject having an ocular wound or fibrosis, the effect of its presence on C3a-activated myofibroblasts was examined. Human corneal keratocytes were stimulated with human C3a over 24 hours and assessed in regard to activated myofibroblasts (Fig. 4). For the detection of activated myofibroblasts aSMA antibodies were used, as well as vimentin antibodies as marker for extracellular matrix. As a reference group, human corneal keratocytes incubated for 24 hours in DMEM growth medium with and without 10% fetal bovine serum (FCS, fetal calf serum) respectively were used. As shown in Fig. 4, C3a 0.1 µg/ml caused significant activation of myofibroblasts (measured by aSMA positive cells, 74±22%) in comparison with the reference group (serumfree: 10±11%; FCS: 16±14%; p < 0.001 and p < 0.001, respectively). Incubation in the presence of C3a and the mouse C5L2 protein fragment resulted in significant decrease of activated myofibroblasts (mC5L2 0.1 µg/ml: 31±13%; mC5L2 0.2 µg/ml: 16±10%; mC5L2 0.3 µg/ml: 21±13%), compared to C3a-activated myofibroblasts (p < 0.001, p < 0.001 and p < 0.001, respectively). Thus, the mouse C5L2 protein fragment was responsible for causing inhibition of myofibroblasts activated by C3a. Bar = Standard error of the mean.

### Example 5

### Mouse C5L2 protein fragment causes inhibition of myofibroblasts activated by C5a

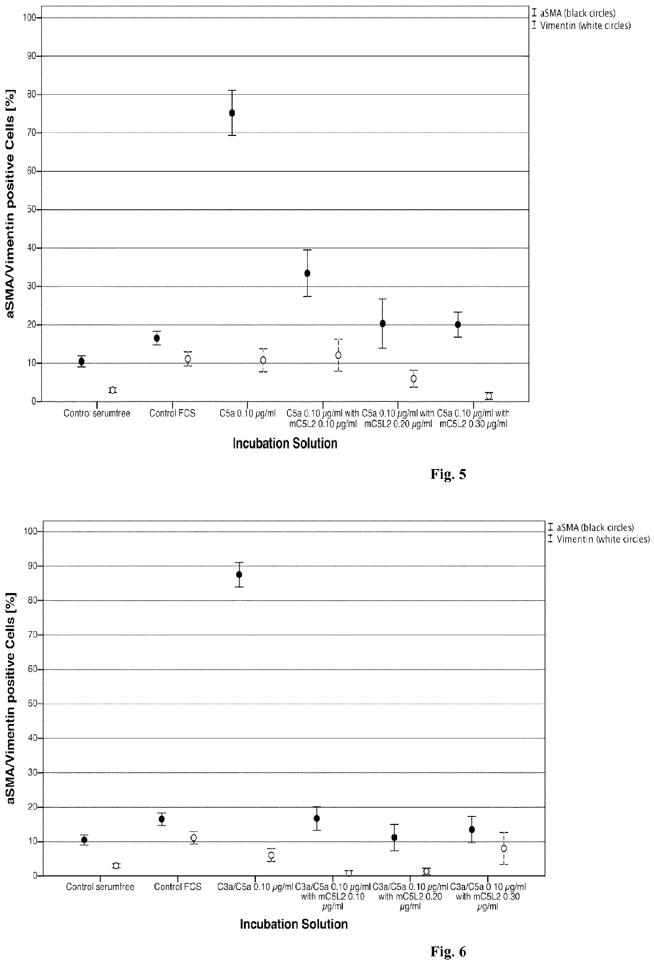

To explore the potential functional role of mouse C5L2 protein fragment (mC5L2), according to SEQ ID No.: 19, in the treatment of a subject having an ocular wound or fibrosis, the effect of its presence on C5a-activated myofibroblasts was examined. Human corneal keratocytes were stimulated with human C5a over 24 hours and assessed in regard to activated myofibroblasts (Fig. 5). For the detection of activated myofibroblasts aSMA antibodies were used, as well as vimentin antibodies as marker for extracellular matrix. As a reference group, human corneal keratocytes incubated for 24 hours in DMEM growth medium with and without 10% fetal bovine serum (FCS, fetal calf serum) respectively were used. As shown in Fig. 5, C5a 0.1 µg/ml caused significant activation of myofibroblasts (measured by aSMA positive cells, 77±23%) in comparison with the reference group (serumfree: 10±11%; FCS: 16±14%; p < 0.001 and p < 0.001, respectively). Incubation in the presence of C5a and the mouse C5L2 protein fragment resulted in significant decrease of activated myofibroblasts (mC5L2 0.1 µg/ml: 33±18%; mC5L2 0.2 µg/ml: 20±19%; mC5L2 0.3 µg/ml: 20±10%), compared to C5a-activated myofibroblasts (p < 0.001, p < 0.001 and p < 0.001, respectively). Thus, the mouse C5L2 protein fragment was responsible for causing inhibition of myofibroblasts activated by C5a. Bar = Standard error of the mean.

### Example 6

### Mouse C5L2 protein fragment causes inhibition of myofibroblasts activated by C5a and C3a

To explore the potential functional role of mouse C5L2 protein fragment (mC5L2), according to SEQ ID No.: 19, in the treatment of a subject having an ocular wound or fibrosis, the effect of its presence on C5a/C3a-activated myofibroblasts was examined. Human corneal keratocytes were stimulated with human C5a and human C3a respectively for 24 hours and assessed in regard to activated myofibroblasts (Fig. 6). For the detection of activated myofibroblasts aSMA antibodies were used, as well as vimentin antibodies as marker for extracellular matrix. As a reference group, human corneal keratocytes incubated for 24 hours in DMEM growth medium with and without 10% fetal bovine serum (FCS, fetal calf serum) respectively were used. As shown in Fig. 6, C5a and C3a, both at a concentration of 0.1 µg/ml, caused significant activation of myofibroblasts (measured by aSMA positive cells, 87±11% respectively) in comparison with the reference group (serumfree: 10±11%; FCS: 16±14%; p < 0.001 and p < 0.001, respectively). Incubation in the presence of C3a, C5a and the mouse C5L2 protein fragment resulted in significant decrease of activated myofibroblasts (mC5L2 0.1 µg/ml: 17±10%; mC5L2 0.2 µg/ml: 11±12%; mC5L2 0.3 µg/ml: 13±11%), compared to C5a- and C3a-activated myofibroblasts (p < 0.001, p < 0.001 and p < 0.001, respectively). Thus, the mouse C5L2 protein fragment was responsible for causing inhibition of myofibroblasts activated by C5a and C3a. Bar = Standard error of the mean.

### Example 7

### The effect of human C5L2 protein fragment concentration on myofibroblasts in the presence of fetal bovine serum

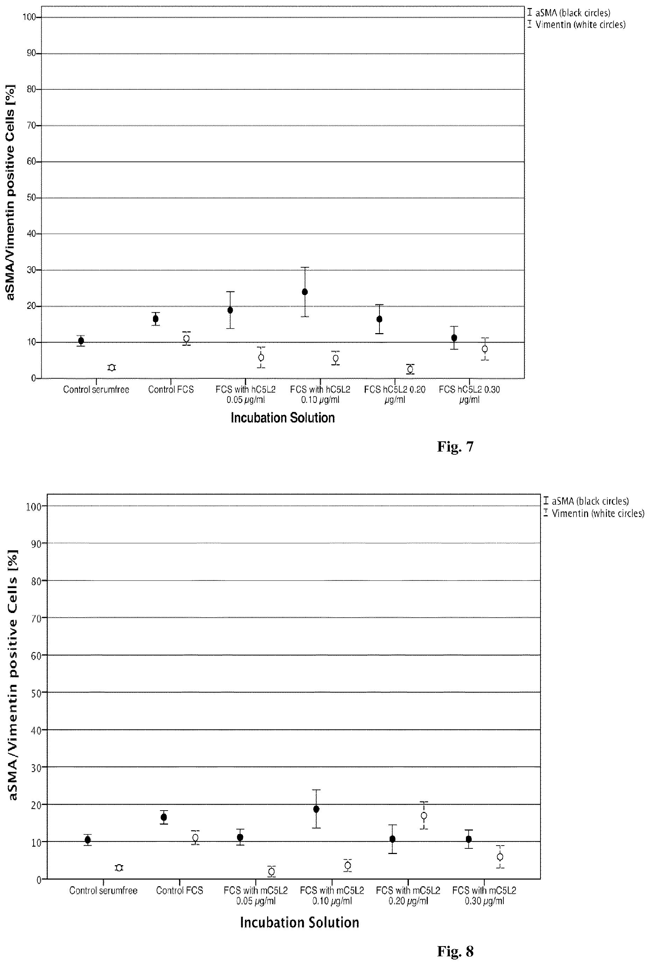

To explore the potential functional role of human C5L2 protein fragment (hC5L2), according to SEQ ID No.: 18, in the treatment of a subject having an ocular wound or fibrosis, the effect of its concentration on myofibroblasts was examined. Human corneal keratocytes were incubated for 24 hours in DMEM growth medium with 10% fetal bovine serum (FCS, fetal calf serum) and human C5L2 protein fragment in different concentrations (Fig. 7). For the detection of activated myofibroblasts aSMA antibodies were used, as well as vimentin antibodies as marker for extracellular matrix. As a reference group, human corneal keratocytes incubated for 24 hours in DMEM growth medium with and without fetal bovine serum respectively were used (serumfree: 10±11%; FCS: 16±14%). As shown in Fig. 7, human C5L2 protein fragment was found to have a slight positive effect on myofibroblasts activation in small concentrations (hC5L2 0.05 µg/ml: 19±15%; hC5L2 0.1 µg/ml: 24±21%; hC5L2 0.2 µg/ml: 16±12%), whereas inhibition of myofibroblasts was observed in higher concentrations (hC5L2 0.3 µg/ml: 11±10%). Yet, compared to 10% FCS incubated human corneal keratocytes, differences remained insignificant (p = 0.554, p = 0.136, p = 0.918 and p = 0.345, respectively).

### Example 8

### The effect of mouse C5L2 protein fragment concentration on myofibroblasts in the presence of fetal bovine serum

To explore the potential functional role of mouse C5L2 protein fragment (mC5L2), according to SEQ ID No.: 19, in the treatment of a subject having an ocular wound or fibrosis, the effect of its concentration on myofibroblasts was examined. Human corneal keratocytes were incubated for 24 hours in DMEM growth medium with 10% fetal bovine serum (FCS, fetal calf serum) and mouse C5L2 protein fragment in different concentrations (Fig. 8). For the detection of activated myofibroblasts aSMA antibodies were used, as well as vimentin antibodies as marker for extracellular matrix. As a reference group, human corneal keratocytes incubated for 24 hours in DMEM growth medium with and without fetal bovine serum respectively were used (serumfree: 10±11%; FCS: 16±14%). As shown in Fig. 8, mouse C5L2 protein fragment was found to have a slightly positive effect on myofibroblasts activation in small concentrations (mC5L2 0.05 µg/ml: 11±6%; mC5L2 0.1 µg/ml: 19±15%; mC5L2 0.2 µg/ml: 11±12%), whereas inhibition of myofibroblasts was observed in higher concentrations (mC5L2 0.3 µg/ml: 11±7%). Yet, compared to 10% FCS incubated human corneal keratocytes, differences remained insignificant (p = 0.101, p = 0.580, p = 0.293 and p = 0.277, respectively).

### Example 9

### The effect of human C5L2 protein fragment concentration on myofibroblasts without fetal bovine serum

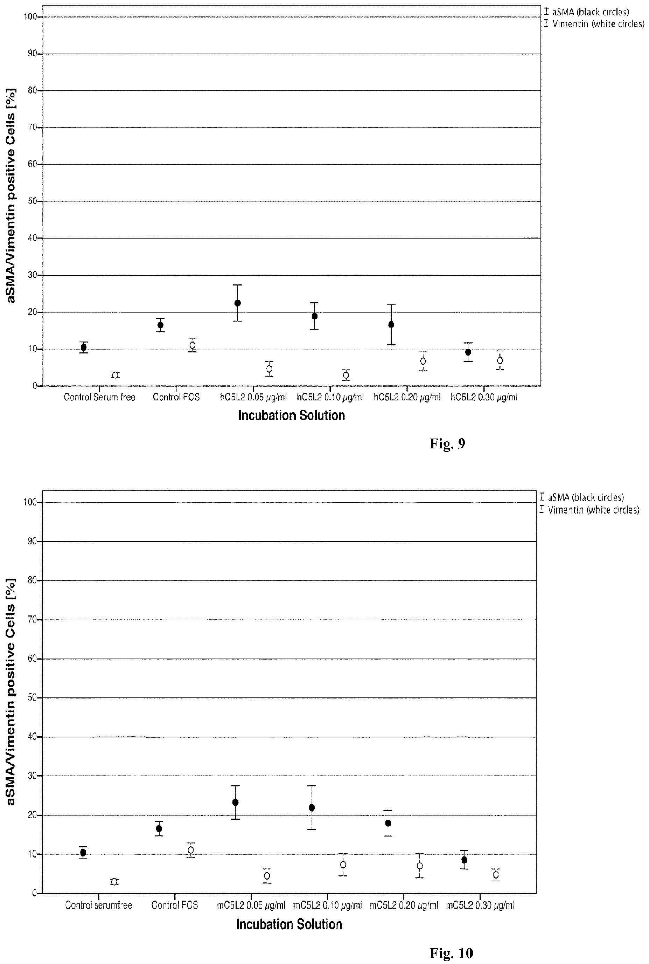

To explore the potential functional role of human C5L2 protein fragment (hC5L2), according to SEQ ID No.: 18, in the treatment of a subject having an ocular wound or fibrosis, the effect of its concentration on myofibroblasts was examined. Human corneal keratocytes were incubated for 24 hours in DMEM growth medium without fetal bovine serum and with human C5L2 protein fragment in different concentrations (Fig. 9). For the detection of activated myofibroblasts aSMA antibodies were used, as well as vimentin antibodies as marker for extracellular matrix. As a reference group, human corneal keratocytes incubated for 24 hours in DMEM growth medium with and without 10% fetal bovine serum (FCS, fetal calf serum) respectively were used (serumfree: 10±11%; FCS: 16±14%). As shown in Fig. 9, human C5L2 protein fragment was found to have a slightly positive effect on myofibroblasts activation, compared to DMEM without FCS incubated human corneal keratocytes (serumfree control), in small concentrations (hC5L2 0.05 µg/ml: 23±15%; hC5L2 0.1 µg/ml: 19±11%; p = 0.005 and p = 0.039, respectively), whereas inhibition of myofibroblasts was observed in higher concentrations and did not reveal a difference to the serumfree control (hC5L2 0.2 µg/ml: 17±16%; hC5L2 0.3 µg/ml: 9±8%; p = 0.150 and p = 0.755, respectively). A list of genes, attained from human corneal keratocytes and generated from a gene expression Clariom S human microarray, that have differing expression levels (fold change: ≥2 or ≤ -2) after 24 hours of incubation with human C5L2 protein fragment 0.3 µg/ml, according to SEQ ID No.: 18, and DMEM growth medium without fetal bovine serum (serumfree control) is shown in Table 8.

### Example 10

### The effect of mouse C5L2 protein fragment concentration on myofibroblasts without fetal bovine serum

To explore the potential functional role of mouse C5L2 protein fragment (mC5L2), according to SEQ ID No.: 19, in the treatment of a subject having an ocular wound or fibrosis, the effect of its concentration on myofibroblasts was examined. Human corneal keratocytes were incubated for 24 hours in DMEM growth medium without fetal bovine serum and with mouse C5L2 protein fragment in different concentrations (Fig. 10). For the detection of activated myofibroblasts aSMA antibodies were used, as well as vimentin antibodies as marker for extracellular matrix. As a reference group, human corneal keratocytes incubated for 24 hours in DMEM growth medium with and without 10% fetal bovine serum (FCS, fetal calf serum) respectively were used (serumfree: 10±11%; FCS: 16±14%). As shown in Fig. 10, mouse C5L2 protein fragment was found to have a slight positive effect on myofibroblasts activation, compared to DMEM without FCS incubated human corneal keratocytes (serumfree control), in small concentrations (mC5L2 0.05 µg/ml: 23±13%; mC5L2 0.1 µg/ml: 22±17%; p = 0.003 and p = 0.009, respectively), whereas inhibition of myofibroblasts was observed in higher concentrations and did not reveal a difference to the serumfree control (hC5L2 0.2 µg/ml: 18±10%; hC5L2 0.3 µg/ml: 9±7%; p = 0.064 and p = 0.647, respectively).

| **Table 2. Differentiation of gene expression of human corneal keratocytes after 24 hours of incubation with human C3a 0.1 µg/ml and serum-free medium (control).** | | | | | | | |
|---|---|---|---|---|---|---|---|
| **Gene Symbol** | **C3a Avg (log2)** | **serumfree Avg (log2)** | **Fold Change** | **Gene Symbol** | **C3a Avg (log2)** | **serumfree Avg (log2)** | **Fold Change** |
| MYO1E | 4,33 | 6,52 | -4,55 | EPB41 L3 | 5,83 | 4,83 | 2 |
| EPHA4 | 3,64 | 5,57 | -3,82 | MED12L | 7,47 | 6,47 | 2 |
| PPM1B | 3,49 | 5,21 | -3,29 | LIMCH1 | 12 | 10,99 | 2 |
| ZNF573 | 5,67 | 7,35 | -3,22 | RP11-93O14.2 | 7,11 | 6,1 | 2,01 |
| JAK2 | 4,95 | 6,46 | -2,85 | SMAD6 | 9,15 | 8,14 | 2,01 |
| RPS3A | 5,5 | 7 | -2,83 | ANKS3 | 8,2 | 7,19 | 2,02 |
| ART1 | 4,66 | 6,09 | -2,71 | SLC27A2 | 6,46 | 5,44 | 2,04 |
| CCBE1 | 4,73 | 6,11 | -2,6 | ANKRD1 | 8,96 | 7,94 | 2,04 |
| TUBGCP3 | 4 | 5,37 | -2,6 | VIPR1 | 7,25 | 6,22 | 2,04 |
| WDR1 | 4,58 | 5,95 | -2,59 | OR4F29 | 6,2 | 5,17 | 2,05 |
| EDA | 4,19 | 5,57 | -2,59 | OR4F16 | 6,2 | 5,17 | 2,05 |
| EXT1; hunera | 8,7 | 10,07 | -2,58 | MX2 | 6,77 | 5,73 | 2,06 |
| SGMS2 | 4,2 | 5,53 | -2,52 | CDH6 | 9,56 | 8,52 | 2,06 |
| C18orf63 | 3,69 | 5,02 | -2,51 | GLB1L | 7,79 | 6,75 | 2,06 |
| WNK2 | 5,95 | 7,26 | -2,47 | HGD | 5,13 | 4,07 | 2,08 |
| NTM | 9,16 | 10,45 | -2,45 | WRB | 5,35 | 4,29 | 2,09 |
| OR12D2 | 3,13 | 4,42 | -2,45 | TECPR2 | 6,82 | 5,76 | 2,1 |
| CYP2R1 | 3,91 | 5,18 | -2,41 | DACT1 | 5,64 | 4,57 | 2,1 |
| SLC44A5 | 4,72 | 5,98 | -2,39 | NCKAP5 | 6,63 | 5,56 | 2,1 |
| SECTM1 | 4,39 | 5,64 | -2,39 | ZBBX | 3,93 | 2,85 | 2,13 |
| MICAL2 | 4,44 | 5,67 | -2,36 | RGS20 | 6,26 | 5,16 | 2,14 |
| BTLA | 3,78 | 5 | -2,34 | ASCL3 | 4,99 | 3,89 | 2,14 |
| IGIP | 5,71 | 6,93 | -2,33 | ZNF502 | 5,78 | 4,68 | 2,15 |
| SCIN | 4,05 | 5,27 | -2,32 | UBE2D3 | 5,1 | 3,99 | 2,15 |
| POGZ | 6,73 | 7,93 | -2,3 | DIRAS3 | 9,25 | 8,13 | 2,17 |
| MXD1 | 4,8 | 5,99 | -2,28 | LYPD6B | 8,76 | 7,64 | 2,18 |
| TOPAZ 1 | 4,11 | 5,3 | -2,28 | MAL2 | 5,7 | 4,57 | 2,19 |
| OR52E8 | 4,22 | 5,4 | -2,28 | INPP5E | 8,16 | 7,03 | 2,19 |
| C18orf65 | 4,19 | 5,37 | -2,27 | SEMA3D | 6,56 | 5,43 | 2,19 |
| AF131215.3 | 4,83 | 6,02 | -2,27 | WNT2 | 7,49 | 6,34 | 2,22 |
| PLGLB2 | 4,29 | 5,46 | -2,26 | TRHDE | 5,17 | 4 | 2,26 |
| ZNF436-AS1 | 4,72 | 5,89 | -2,25 | RBKS | 5,85 | 4,67 | 2,26 |
| TMEM14EP | 2,9 | 4,06 | -2,23 | TAS2R50 | 4,64 | 3,47 | 2,26 |
| HDLBP | 4,94 | 6,09 | -2,23 | THSD4 | 5,97 | 4,8 | 2,26 |
| OR52E1 | 3,82 | 4,96 | -2,22 | DMD | 8,61 | 7,39 | 2,33 |
| TMEM204 | 4,02 | 5,17 | -2,21 | CHTF8 | 5,57 | 4,34 | 2,34 |
| EFHC2 | 3,14 | 4,28 | -2,21 | KIRREL3 | 5,29 | 4,05 | 2,36 |
| FEZF2 | 4,4 | 5,53 | -2,2 | ADAM28 | 5,08 | 3,84 | 2,36 |
| TAF1 | 4,62 | 5,76 | -2,19 | FAM46C | 5,68 | 4,42 | 2,4 |
| KLHDC4 | 4,91 | 6,04 | -2,19 | TINAG | 5,14 | 3,87 | 2,4 |
| ITGB2 | 3,95 | 5,08 | -2,18 | CDNF | 5,34 | 4,07 | 2,41 |
| ARR3 | 4,39 | 5,51 | -2,17 | CHRM3 | 6,97 | 5,7 | 2,42 |
| C1QTNF6 | 5,09 | 6,2 | -2,17 | RPS6KA5 | 8,17 | 6,86 | 2,47 |
| TTC39B | 4,4 | 5,51 | -2,16 | IGF2 | 7,06 | 5,72 | 2,54 |
| FOXO1 | 3,45 | 4,56 | -2,16 | PTGFRN | 8,86 | 7,5 | 2,56 |
| DOCK 10 | 5,49 | 6,58 | -2,13 | SPIB | 5,16 | 3,79 | 2,6 |
| DUX4 | 4,31 | 5,4 | -2,13 | OSBPL1A | 4,56 | 3,18 | 2,61 |
| RAB39B | 3,21 | 4,3 | -2,13 | ANKRD 18B | 6,05 | 4,65 | 2,63 |
| BIRC3 | 4,03 | 5,12 | -2,12 | FLOT2 | 6,49 | 5,08 | 2,66 |
| SF1 | 7,68 | 8,76 | -2,12 | ZNF546 | 5,66 | 4,24 | 2,68 |
| KIRREL3 | 6,66 | 7,74 | -2,11 | NME5 | 5,58 | 4,14 | 2,72 |
| COL6A2 | 4,5 | 5,57 | -2,1 | PDE1C | 11,56 | 10,1 | 2,74 |
| TFRC | 3,54 | 4,6 | -2,09 | SERPINB2 | 8,32 | 6,85 | 2,78 |
| PPEF2 | 4,53 | 5,58 | -2,08 | RFX4 | 5,77 | 4,29 | 2,81 |
| ST8SIA1 | 3,86 | 4,92 | -2,08 | SEMA5A | 7,66 | 6,11 | 2,92 |
| UTS2B | 4,78 | 5,83 | -2,06 | RGS7BP | 6,83 | 5,27 | 2,94 |
| HSFX1 | 5,77 | 6,81 | -2,06 | IL6 | 9,52 | 7,86 | 3,15 |
| LYZL6 | 3,42 | 4,47 | -2,06 | RARB | 6,21 | 4,52 | 3,23 |
| MBOAT2 | 4,97 | 6,01 | -2,06 | ANKRD44 | 5,66 | 3,93 | 3,32 |
| CHAC1 | 10,11 | 11,15 | -2,05 | SULF1 | 11,88 | 9,32 | 5,89 |
| TPD52 | 8,55 | 9,58 | -2,04 | | | | |
| XRCC5 | 6,8 | 7,83 | -2,04 | | | | |
| RBMS1 | 4,69 | 5,72 | -2,04 | | | | |
| IFNA7 | 3,2 | 4,22 | -2,03 | | | | |
| SCAPER | 3,66 | 4,68 | -2,03 | | | | |
| LINGO4 | 4,32 | 5,34 | -2,03 | | | | |
| ANKRD36 | 6,9 | 7,92 | -2,03 | | | | |
| C5orf66 | 5,1 | 6,12 | -2,03 | | | | |
| SQSTM1 | 3,88 | 4,9 | -2,02 | | | | |
| LTBP4 | 8,25 | 9,26 | -2,02 | | | | |
| NEK5 | 4,09 | 5,11 | -2,02 | | | | |
| MYOID | 5,96 | 6,98 | -2,02 | | | | |
| ANK2 | 5,05 | 6,06 | -2,02 | | | | |
| HCRP1 | 3,59 | 4,6 | -2,01 | | | | |
| SLC38A9 | 4,82 | 5,83 | -2,01 | | | | |
| SUMO4 | 7,16 | 8,17 | -2,01 | | | | |
| KSR2 | 4,19 | 5,2 | -2,01 | | | | |
| PILRB | 4,1 | 5,11 | -2,01 | | | | |
| STK32C | 7,67 | 8,67 | -2 | | | | |
| SPIRE2 | 5,63 | 6,63 | -2 | | | | |
| DCST1 | 5,33 | 6,34 | -2 | | | | |
| UNC13A | 3,8 | 4,8 | -2 | | | | |

| **Table 3. Differentiation of gene expression of human corneal keratocytes after 24 hours of incubation with human C5a 0.1 µg/ml and serum-free medium (control).** | | | | | | | |
|---|---|---|---|---|---|---|---|
| **Gene Symbol** | **C5a Avg (log2)** | **serumfree Avg (log2)** | **Fold Change** | **Gene Symbol** | **C5a Avg (log2)** | **serumfree Avg (log2)** | **Fold Change** |
| CAMKMT | 3,55 | 5,74 | -4,56 | CLEC4M | 6,79 | 5,78 | 2,01 |
| RAB28 | 4,55 | 6,29 | -3,34 | ZNF582-AS1 | 5,45 | 4,43 | 2,02 |
| JAK2 | 4,88 | 6,46 | -3 | TMEM212 | 4,34 | 3,32 | 2,03 |
| PKP4 | 5,6 | 7,17 | -2,96 | TDRD12 | 4,17 | 3,15 | 2,03 |
| CYP2R1 | 3,66 | 5,18 | -2,87 | CDON | 8,32 | 7,3 | 2,04 |
| BACH2 | 2,98 | 4,45 | -2,77 | MTNR1A | 5,48 | 4,45 | 2,04 |
| PLEKHA6 | 4,57 | 6,03 | -2,75 | SORCS1 | 5,97 | 4,94 | 2,04 |
| EXT1; hunera | 8,62 | 10,07 | -2,72 | RPS6KA5 | 7,9 | 6,86 | 2,04 |
| LCORL | 4,2 | 5,61 | -2,65 | TSGA10IP | 7,6 | 6,57 | 2,04 |
| PSMB8-AS1 | 5,38 | 6,78 | -2,64 | CSPG5 | 5,61 | 4,57 | 2,06 |
| CSMD1 | 3,59 | 4,97 | -2,6 | SLC22A18AS | 7,81 | 6,76 | 2,06 |
| RPS3A | 5,63 | 7 | -2,58 | OR9K2 | 8,03 | 6,96 | 2,09 |
| OR5F1 | 4,32 | 5,66 | -2,55 | ERV3-1 | 8 | 6,93 | 2,1 |
| MTFP1 | 4,72 | 6,04 | -2,5 | PCP2 | 7,41 | 6,33 | 2,12 |
| CD53 | 3,61 | 4,91 | -2,47 | PTPRR | 5,11 | 4,01 | 2,14 |
| LSM6 | 5,63 | 6,91 | -2,43 | GAGE2D | 4,81 | 3,72 | 2,14 |
| TBC1D3 | 8,02 | 9,29 | -2,4 | IFNA8 | 6,22 | 5,12 | 2,14 |
| TAAR2 | 3,92 | 5,18 | -2,4 | TMEM179 | 5,48 | 4,37 | 2,15 |
| POGZ | 5,47 | 6,73 | -2,39 | PCSK2 | 4,47 | 3,36 | 2,15 |
| RAD54L | 5,37 | 6,62 | -2,38 | WDR78 | 6,28 | 5,16 | 2,17 |
| MARCH1 | 3,46 | 4,71 | -2,37 | FLOT2 | 6,22 | 5,08 | 2,2 |
| ITGB6 | 4 | 5,24 | -2,36 | GOLGA8N | 6,57 | 5,41 | 2,22 |
| TBC1D3H | 8,9 | 10,14 | -2,35 | IFIH1 | 8,31 | 7,15 | 2,23 |
| DACH1 | 3,3 | 4,52 | -2,33 | MPZL3 | 8,2 | 7,04 | 2,23 |
| PCDHB1 | 3,76 | 4,98 | -2,33 | THEM5 | 6,47 | 5,3 | 2,25 |
| NR2C2 | 5,61 | 6,81 | -2,3 | FNDC7 | 4,95 | 3,78 | 2,25 |
| SF1 | 7,56 | 8,76 | -2,3 | AACSP1 | 5,81 | 4,63 | 2,27 |
| TRIM10 | 5,42 | 6,6 | -2,26 | METTL7B | 7,45 | 6,26 | 2,29 |
| EPHA4 | 4,56 | 5,73 | -2,25 | SELL | 4,79 | 3,6 | 2,29 |
| TTC39B | 4,35 | 5,51 | -2,24 | RAB11A | 4,57 | 3,36 | 2,31 |
| LGI2 | 4,2 | 5,36 | -2,24 | INTS 1 | 5,7 | 4,48 | 2,33 |
| TPK1 | 4,62 | 5,78 | -2,24 | RBKS | 5,91 | 4,67 | 2,36 |
| NPL | 3,48 | 4,64 | -2,23 | PLCB1 | 5,66 | 4,42 | 2,36 |
| PNPLA7 | 3,19 | 4,34 | -2,21 | ENKD1 | 7,33 | 6,09 | 2,36 |
| CCDC84 | 8,31 | 9,44 | -2,2 | OR8H2 | 5,07 | 3,82 | 2,37 |
| TAS2R19 | 4,08 | 5,22 | -2,19 | KCNN3 | 5,56 | 4,24 | 2,51 |
| C16orf72 | 5,28 | 6,41 | -2,19 | TINAG | 5,23 | 3,87 | 2,55 |
| HDLBP | 4,97 | 6,09 | -2,17 | DLK1 | 5,27 | 3,91 | 2,56 |
| CLASP2 | 4,46 | 5,57 | -2,17 | PDK4 | 4,72 | 3,25 | 2,77 |
| ANXA2R | 6,86 | 7,97 | -2,17 | CCDC173 | 5,72 | 4,24 | 2,79 |
| SLC44A5 | 4,86 | 5,98 | -2,17 | HBB | 5,99 | 4,47 | 2,87 |
| XRCC5 | 7,57 | 8,68 | -2,16 | LAP3 | 5,87 | 3,75 | 4,36 |
| HESX1 | 4,6 | 5,7 | -2,15 | | | | |
| EXT1; spawla | 6,42 | 7,53 | -2,15 | | | | |
| AKR1C8P | 3,84 | 4,94 | -2,15 | | | | |
| ATP13A3 | 5,33 | 6,43 | -2,15 | | | | |
| OXCT2P1 | 5,72 | 6,82 | -2,14 | | | | |
| ZBTB9 | 8,17 | 9,27 | -2,14 | | | | |
| TMEM236 | 3,75 | 4,83 | -2,12 | | | | |
| CYP39A1 | 3,35 | 4,43 | -2,12 | | | | |
| SLC17A1 | 3,95 | 5,03 | -2,11 | | | | |
| STK33 | 3,45 | 4,52 | -2,1 | | | | |
| GALNTL5 | 3,87 | 4,94 | -2,1 | | | | |
| TAS2R31 | 7,49 | 8,56 | -2,09 | | | | |
| CPLX4 | 4,95 | 6,01 | -2,09 | | | | |
| CC2D2A | 3,31 | 4,37 | -2,09 | | | | |
| MXD1 | 4,93 | 5,99 | -2,08 | | | | |
| MS4A4E | 4,65 | 5,7 | -2,07 | | | | |
| TNKS | 5,98 | 7,03 | -2,06 | | | | |
| KRT23 | 4,56 | 5,6 | -2,04 | | | | |
| OR12D2 | 3,39 | 4,42 | -2,04 | | | | |
| HSFX2 | 6,64 | 7,67 | -2,04 | | | | |
| TJP1 | 4,59 | 5,61 | -2,04 | | | | |
| PLGLB2 | 4,44 | 5,46 | -2,04 | | | | |
| LRRC20 | 5,23 | 6,25 | -2,03 | | | | |
| KIRREL3 | 6,72 | 7,74 | -2,02 | | | | |
| PAQR6 | 4,86 | 5,87 | -2,02 | | | | |
| KDM4C | 5,62 | 6,63 | -2,02 | | | | |
| PTAFR | 4,49 | 5,5 | -2,02 | | | | |
| NEK5 | 4,09 | 5,11 | -2,02 | | | | |
| ZNF721 | 7,83 | 8,84 | -2,02 | | | | |
| PYY | 5,03 | 6,04 | -2,02 | | | | |
| TBC1D3G | 7,84 | 8,85 | -2,01 | | | | |
| TCERG1 | 4,02 | 5,03 | -2,01 | | | | |
| ZNF436-AS1 | 4,88 | 5,89 | -2 | | | | |
| ERAS | 5,21 | 6,22 | -2 | | | | |
| NAIP | 6,31 | 7,31 | -2 | | | | |
| OR5T1 | 4,38 | 5,38 | -2 | | | | |
| SLC24A2 | 4,49 | 3,48 | 2 | | | | |

| **Table 4. Differentiation of gene expression of human corneal keratocytes after 24 hours of incubation with human C3a/C5a 0.1 µg/ml and serum-free medium.** | | | | | | | |
|---|---|---|---|---|---|---|---|
| **Gene Symbol** | **C3a/C5a Avg (log2)** | **serumfree Avg (log2)** | **Fold Change** | **Gene Symbol** | **C3a/C5a Avg (log2)** | **serumfree Avg (log2)** | **Fold Change** |
| OCRL | 4,57 | 6,47 | -3,71 | DZANK1 | 5,76 | 4,75 | 2,01 |
| TJP1 | 3,98 | 5,61 | -3,1 | ADAM18 | 5,11 | 4,11 | 2,01 |
| MYO1E | 4,9 | 6,52 | -3,07 | RGS7BP | 6,29 | 5,27 | 2,02 |
| TAF1 | 4,2 | 5,76 | -2,93 | KCNAB2 | 9,53 | 8,51 | 2,02 |
| HYOU 1 | 3,64 | 5,19 | -2,93 | TCAP | 9,99 | 8,97 | 2,02 |
| SYT1 | 5,24 | 6,79 | -2,93 | PVALB | 5,61 | 4,59 | 2,03 |
| SCAPER | 3,23 | 4,68 | -2,72 | HOXB5 | 6,84 | 5,82 | 2,03 |
| HK2 | 7,68 | 9,12 | -2,72 | SPATA45 | 5,33 | 4,31 | 2,03 |
| NTM | 9,05 | 10,45 | -2,63 | MS4A5 | 5,87 | 4,85 | 2,03 |
| CYP2R1 | 3,8 | 5,18 | -2,61 | SYT12 | 5,42 | 4,4 | 2,03 |
| CST9L | 3,64 | 5,01 | -2,58 | TACSTD2 | 5,08 | 4,06 | 2,03 |
| LTBP2 | 6,22 | 7,58 | -2,56 | NME5 | 5,16 | 4,14 | 2,04 |
| OR10AG1 | 6,05 | 7,38 | -2,51 | IL1B | 10,45 | 9,42 | 2,04 |
| MAGEE1 | 4,9 | 6,22 | -2, 5 | OR2B2 | 4,36 | 3,34 | 2,04 |
| EPHA4 | 4,27 | 5,57 | -2,48 | CDH6 | 9,55 | 8,52 | 2,04 |
| PLEKHA6 | 4,76 | 6,03 | -2,42 | ATP10A | 5,43 | 4,4 | 2,05 |
| MXD1 | 4,72 | 5,99 | -2,4 | EPB41 | 6,82 | 5,79 | 2,05 |
| POGZ | 6,67 | 7,93 | -2,4 | ATP4B | 5,04 | 4,01 | 2,05 |
| LAPTM4A | 4,42 | 5,67 | -2,39 | KCNN3 | 5,27 | 4,24 | 2,05 |
| ARPP21 | 3,98 | 5,23 | -2,38 | CXCL8 | 7,26 | 6,22 | 2,06 |
| STC1 | 10,62 | 11,86 | -2,37 | PATE1 | 5,78 | 4,74 | 2,06 |
| CADPS | 3,16 | 4,4 | -2,36 | FLG | 4,62 | 3,57 | 2,06 |
| MTFP1 | 4,8 | 6,04 | -2,35 | NTF3 | 9,78 | 8,74 | 2,06 |
| YEATS2 | 3,24 | 4,47 | -2,35 | ST6GAL1 | 7,55 | 6,49 | 2,08 |
| ZNF512 | 4,99 | 6,21 | -2,33 | TES | 11,25 | 10,19 | 2,08 |
| EDA | 4,37 | 5,57 | -2,3 | LAP 3 | 4,81 | 3,75 | 2,08 |
| RAB2A | 4,53 | 5,73 | -2,29 | LINC01588 | 5,54 | 4,48 | 2,09 |
| OR5F1 | 4,47 | 5,66 | -2,29 | CNTNAP3P2 | 7,46 | 6,39 | 2,09 |
| PTPRB | 6,28 | 7,48 | -2,29 | CHTF8 | 5,41 | 4,34 | 2,09 |
| BNIP3 | 13,79 | 14,97 | -2,27 | RASAL3 | 8,24 | 7,17 | 2,1 |
| KIRREL3 | 6,55 | 7,74 | -2,27 | GPRC5B | 11,18 | 10,1 | 2,12 |
| CEP97 | 6,16 | 7,34 | -2,27 | ANO9 | 5,04 | 3,96 | 2,12 |
| GMDS | 6,87 | 8,04 | -2,26 | SPRR3 | 5,24 | 4,15 | 2,13 |
| ZNF165 | 3,98 | 5,15 | -2,25 | TTC21A | 6,56 | 5,47 | 2,13 |
| VCAN | 9,69 | 10,86 | -2,25 | F2RL1 | 7,64 | 6,54 | 2,14 |
| TPD52 | 8,42 | 9,58 | -2,23 | RBM26 | 6,59 | 5,49 | 2,14 |
| TEF | 5,97 | 7,12 | -2,23 | C7orf69 | 7,73 | 6,63 | 2,14 |
| GPR173 | 3,83 | 4,98 | -2,23 | ZNF527 | 6,05 | 4,95 | 2,14 |
| FAM151B | 4,88 | 6,02 | -2,2 | ICA1 | 4,71 | 3,61 | 2,14 |
| PCDHB13 | 5,37 | 6,51 | -2,2 | ITGB4 | 6,02 | 4,91 | 2,15 |
| ZBTB9 | 8,14 | 9,27 | -2,2 | SNX29P2 | 8,01 | 6,9 | 2,15 |
| TMEM204 | 4,03 | 5,17 | -2,2 | ESAM | 5,93 | 4,83 | 2,15 |
| THSD4 | 8,41 | 9,55 | -2,2 | MRAS | 6,42 | 5,31 | 2,16 |
| MYOID | 5,85 | 6,98 | -2,19 | EPB41L3 | 5,94 | 4,83 | 2,16 |
| SERTAD3 | 7,52 | 8,65 | -2,18 | EPHA7 | 6,18 | 5,07 | 2,16 |
| PCDHB1 | 3,86 | 4,98 | -2,18 | CDRT1 | 5,73 | 4,61 | 2,17 |
| SGMS2 | 4,41 | 5,53 | -2,17 | HLA-DQB2 | 7,85 | 6,73 | 2,17 |
| DNM3 | 3,83 | 4,95 | -2,17 | DDX11 | 6,55 | 5,43 | 2,18 |
| WNK2 | 6,15 | 7,26 | -2,16 | ASCL3 | 5,01 | 3,89 | 2,18 |
| AGR2 | 3,73 | 4,84 | -2,15 | RARRES 1 | 9,61 | 8,48 | 2,18 |
| NLRP3 | 3,61 | 4,72 | -2,15 | SSPN | 6,53 | 5,4 | 2,19 |
| ITGB6 | 4,13 | 5,24 | -2,15 | LYPD6 | 7,01 | 5,88 | 2,19 |
| LINC01537 | 3,86 | 4,96 | -2,15 | SERPINA4 | 4,88 | 3,75 | 2,19 |
| SKP2 | 5,3 | 6,41 | -2,15 | PHOSPHO1 | 5,76 | 4,62 | 2,19 |
| EPHA4 | 4,63 | 5,73 | -2,15 | C1orf198 | 10,42 | 9,28 | 2,2 |
| RASSF9 | 3,26 | 4,37 | -2,15 | DACT1 | 5,72 | 4,57 | 2,21 |
| NAIP | 5,78 | 6,88 | -2,14 | SPNS3 | 8,1 | 6,96 | 2,21 |
| NLRP9 | 4,37 | 5,46 | -2,13 | AIM1L | 6,35 | 5,2 | 2,22 |
| CSTA | 5,21 | 6,29 | -2,13 | TAS2R50 | 4,62 | 3,47 | 2,23 |
| DGKD | 5,05 | 6,13 | -2,11 | OR4F6 | 4,36 | 3,21 | 2,23 |
| PIP4K2A | 4,4 | 5,48 | -2,11 | LAIR1 | 5,33 | 4,17 | 2,23 |
| OR5T1 | 4,31 | 5,38 | -2,11 | IL7 | 5,07 | 3,91 | 2,24 |
| KLF17 | 3,78 | 4,85 | -2,11 | KCNK17 | 7,92 | 6,76 | 2,24 |
| RPS3A | 5,92 | 7 | -2,1 | RAB7B | 7,87 | 6,7 | 2,24 |
| TTC25 | 5,47 | 6,54 | -2,09 | ANKRD18B | 5,83 | 4,65 | 2,25 |
| CSMD1 | 3,91 | 4,97 | -2,08 | C10orf10 | 7,75 | 6,57 | 2,26 |
| DNASE1 | 5,08 | 6,14 | -2,08 | BLK | 7,84 | 6,65 | 2,27 |
| CCDC159 | 4,85 | 5,9 | -2,08 | WNT2 | 7,53 | 6,34 | 2,27 |
| DDIT4 | 11 | 12,06 | -2,08 | ITGB8 | 6,64 | 5,45 | 2,28 |
| QRICH1 | 6,44 | 7,49 | -2,08 | SORCS1 | 6,13 | 4,94 | 2,28 |
| HDLBP | 5,04 | 6,09 | -2,08 | THEM5 | 6,51 | 5,3 | 2,3 |
| MYRIP | 7,02 | 8,08 | -2,07 | DEFB105B | 5,28 | 4,08 | 2,3 |
| NLRP1 | 8 | 9,05 | -2,07 | EVPLL | 5,74 | 4,53 | 2,3 |
| BIRC3 | 4,07 | 5,12 | -2,06 | LILRB3 | 4,9 | 3,69 | 2,31 |
| BACH2 | 3,41 | 4,45 | -2,06 | ZSCAN31 | 7,19 | 5,97 | 2,32 |
| PEX5L | 4,88 | 5,92 | -2,06 | OR52E6 | 5,52 | 4,31 | 2,33 |
| KDM4C | 5,59 | 6,63 | -2,05 | PDE1C | 11,34 | 10,1 | 2,36 |
| PITPNM2 | 5,51 | 6,55 | -2,05 | UGT2B10 | 4,4 | 3,14 | 2,39 |
| ARHGAP15 | 3,44 | 4,47 | -2,04 | RGS20 | 6,43 | 5,16 | 2,41 |
| VLDLR | 8,63 | 9,66 | -2,04 | HTR5A | 5,16 | 3,89 | 2,42 |
| AP1S1 | 5,56 | 6,59 | -2,04 | PABPC4L | 7,6 | 6,32 | 2,42 |
| EFCAB13 | 5,43 | 6,45 | -2,04 | CSPG5 | 5,85 | 4,57 | 2,43 |
| PRKAA2 | 5,92 | 6,95 | -2,03 | CYTH4 | 6,54 | 5,26 | 2,44 |
| ZNF385D | 5,68 | 6,7 | -2,02 | TMEM212 | 4,61 | 3,32 | 2,45 |
| SRP72 | 4,69 | 5,71 | -2,02 | NCKAP5 | 6,87 | 5,56 | 2,48 |
| | | | | LMOD3 | 4,87 | 3,56 | 2,48 |
| | | | | MS4A12 | 5,93 | 4,61 | 2,5 |
| | | | | Sep 14 | 5,25 | 3,91 | 2,53 |
| | | | | UBE2D3 | 5,34 | 3,99 | 2,55 |
| | | | | SEMA5A | 7,47 | 6,11 | 2,56 |
| | | | | NGB | 5,39 | 4,02 | 2,59 |
| | | | | PDK4 | 4,65 | 3,25 | 2,65 |
| | | | | PTGFRN | 8,91 | 7,5 | 2,65 |
| | | | | TAL1 | 5,78 | 4,37 | 2,65 |
| | | | | ADAM28 | 5,25 | 3,84 | 2,66 |
| | | | | IGF2 | 7,14 | 5,72 | 2,67 |
| | | | | RARB | 5,96 | 4,52 | 2,72 |
| | | | | SERPINB2 | 8,33 | 6,85 | 2,78 |
| | | | | PADI4 | 5,84 | 4,35 | 2,81 |
| | | | | C8orf46 | 6,35 | 4,8 | 2,91 |
| | | | | FLOT2 | 6,63 | 5,08 | 2,92 |
| | | | | IL6 | 9,96 | 7,86 | 4,28 |
| | | | | SULF1 | 12,24 | 9,32 | 7,57 |

| **Table 5. Differentiation of gene expression of human corneal keratocytes after 24 hours of incubation with human C3a 0.1 µg/ml and human C3a 0.1 µg/ml with human C5L2 0.3 µg/ml,** | | | | | | | |
|---|---|---|---|---|---|---|---|
| **Gene Symbol** | **C3a Avg (log2)** | **C3a/C5L2 Avg (log2)** | **Fold Change** | **Gene Symbol** | **C3a Avg (log2)** | **C3a/C5L2 Avg (log2)** | **Fold Change** |
| SLC38A9 | 4,82 | 6,65 | -3,56 | NME5 | 5,58 | 4,58 | 2 |
| ART 1 | 4,66 | 6,4 | -3,35 | SNRPD2P2 | 5,36 | 4,36 | 2,01 |
| RFX3 | 5,18 | 6,84 | -3,15 | FAM46C | 5,68 | 4,67 | 2,02 |
| TUBGCP3 | 4 | 5,62 | -3,09 | GPR157 | 8,27 | 7,25 | 2,02 |
| WDR1 | 4,58 | 6,15 | -2,97 | SAR1B | 5,25 | 4,23 | 2,03 |
| CLCNKB | 3,79 | 5,36 | -2,97 | AKR1C8P | 4,97 | 3,95 | 2,03 |
| BIRC3 | 4,03 | 5,59 | -2,94 | ZNF812P | 5,46 | 4,43 | 2,04 |
| POLE | 4,69 | 6,23 | -2,9 | KCNAB1 | 5,62 | 4,59 | 2,04 |
| HIPK 1 | 5,5 | 7,02 | -2,87 | RFX4 | 5,77 | 4,73 | 2,06 |
| IGIP | 5,71 | 7,23 | -2,86 | TRAF1 | 6,2 | 5,15 | 2,07 |
| UBR3 | 3,41 | 4,91 | -2,83 | BCL7A | 5,01 | 3,95 | 2,09 |
| JAK2 | 4,95 | 6,38 | -2,69 | ZFPM2 | 5,55 | 4,48 | 2,1 |
| RGS16 | 4,34 | 5,7 | -2,57 | HRH1 | 7,06 | 5,98 | 2,1 |
| CYLC2 | 4,53 | 5,86 | -2,52 | TAS2R50 | 4,64 | 3,57 | 2,1 |
| SIDT1 | 4,78 | 6,1 | -2,49 | OTUD6B | 5,5 | 4,43 | 2,11 |
| SLC23A3 | 5,08 | 6,4 | -2,49 | ANKRD44 | 5,66 | 4,58 | 2,11 |
| STON2 | 4,08 | 5,4 | -2,49 | MYH9 | 5,9 | 4,81 | 2,13 |
| MPO | 3,65 | 4,96 | -2,48 | KPNA7 | 5,05 | 3,96 | 2,13 |
| ZNF436-AS1 | 4,72 | 6,02 | -2,48 | SPN | 5,66 | 4,57 | 2,14 |
| CAPN3 | 4,2 | 5,49 | -2,43 | TFAP2C | 5,57 | 4,47 | 2,15 |
| TK2 | 4,61 | 5,87 | -2,39 | CTAGE5 | 7,56 | 6,46 | 2,15 |
| FMN1 | 6,07 | 7,32 | -2,38 | SMIM14 | 9,25 | 8,14 | 2,16 |
| OR13G1 | 4,18 | 5,43 | -2,38 | PLEKHA6 | 5,12 | 4 | 2,18 |
| RFC3 | 5,48 | 6,73 | -2,37 | TCEANC | 4,76 | 3,62 | 2,2 |
| S100A14 | 5,03 | 6,27 | -2,37 | ITGB6 | 4,82 | 3,68 | 2,2 |
| PTPN13 | 5 | 6,21 | -2,31 | MS4A7 | 5,04 | 3,89 | 2,21 |
| ERAP1 | 3,59 | 4,8 | -2,3 | PPP4R4 | 5,03 | 3,88 | 2,22 |
| XKR9 | 4,64 | 5,81 | -2,25 | SIGLEC 15 | 5,78 | 4,6 | 2,27 |
| FCHO1 | 3,99 | 5,15 | -2,23 | ZNF846 | 7,41 | 6,22 | 2,28 |
| B3GALT4 | 4,67 | 5,82 | -2,23 | SPOCD 1 | 9,99 | 8,73 | 2,39 |
| LRRIQ3 | 3,96 | 5,12 | -2,22 | CCDC79 | 5,29 | 4 | 2,43 |
| PRND | 4,46 | 5,61 | -2,22 | A2BP1 | 4,64 | 3,24 | 2,64 |
| TNFRSF25 | 7,59 | 8,73 | -2,21 | OR4F29 | 6,2 | 4,67 | 2,89 |
| RDH5 | 5,82 | 6,95 | -2,19 | OR4F16 | 6,2 | 4,67 | 2,89 |
| MYRFL | 4,53 | 5,65 | -2,17 | TRHDE | 5,17 | 3,6 | 2,99 |
| KCNK1 | 4,15 | 5,27 | -2,17 | | | | |
| LINC01559 | 4,64 | 5,76 | -2,17 | | | | |
| EPB42 | 4,1 | 5,22 | -2,17 | | | | |
| KLRB 1 | 3,91 | 5,02 | -2,16 | | | | |
| CNTNAP3B | 6,16 | 7,26 | -2,15 | | | | |
| HGSNAT | 4,97 | 6,06 | -2,13 | | | | |
| PCSK1 | 4,14 | 5,22 | -2,12 | | | | |
| RIMS2 | 3,53 | 4,62 | -2,12 | | | | |
| PTRH1 | 6,1 | 7,18 | -2,11 | | | | |
| STK32C | 7,67 | 8,75 | -2,11 | | | | |
| EXT1; hunera | 8,7 | 9,77 | -2,11 | | | | |
| GAS2 | 3,2 | 4,27 | -2,1 | | | | |
| SMAD7 | 6,04 | 7,11 | -2,09 | | | | |
| GPR84 | 3,16 | 4,22 | -2,09 | | | | |
| OR2T3 | 6,18 | 7,24 | -2,08 | | | | |
| ASB17 | 3,93 | 4,98 | -2,08 | | | | |
| EFHC2 | 3,14 | 4,2 | -2,08 | | | | |
| DDC | 4,56 | 5,61 | -2,08 | | | | |
| ALPK3 | 4,02 | 5,07 | -2,07 | | | | |
| BSX | 4,33 | 5,38 | -2,07 | | | | |
| PPM1B | 3,49 | 4,53 | -2,06 | | | | |
| DOCK2 | 4,74 | 5,78 | -2,06 | | | | |
| PTK2 | 5,38 | 6,42 | -2,05 | | | | |
| ANKRD18A | 4,24 | 5,26 | -2,04 | | | | |
| CCBE1 | 4,73 | 5,76 | -2,04 | | | | |
| SPATA32 | 4,63 | 5,66 | -2,03 | | | | |
| TOPAZ1 | 4,11 | 5,13 | -2,03 | | | | |
| CALCR | 3,81 | 4,83 | -2,03 | | | | |
| RSPO2 | 4,12 | 5,14 | -2,03 | | | | |
| PCSK2 | 3,34 | 4,36 | -2,03 | | | | |
| TAS1R2 | 4,43 | 5,44 | -2,03 | | | | |
| ZNF396 | 3,74 | 4,75 | -2,02 | | | | |
| ZNF573 | 5,67 | 6,68 | -2,02 | | | | |
| RGSL1 | 3,35 | 4,37 | -2,02 | | | | |
| PPP2R2B | 4,89 | 5,89 | -2,01 | | | | |
| FABP6 | 4 | 5 | -2 | | | | |
| STPG2 | 3,35 | 4,35 | -2 | | | | |
| SLC15A1 | 3,99 | 4,99 | -2 | | | | |

| **Table 6. Differentiation of gene expression of human corneal keratocytes after 24 hours of incubation with human C5a 0.1 µg/ml and human C5a 0.1 µg/ml with human C5L2 0.3** µg/ml. | | | | | | | |
|---|---|---|---|---|---|---|---|
| **Gene Symbol** | **C5a Avg (log2)** | **C5a/C5L2 Avg (log2)** | **Fold Change** | **Gene Symbol** | **C5a Avg (log2)** | **C5a/C5L2 Avg (log2)** | **Fold Change** |
| HNRNPK | 5,53 | 7,32 | -3,46 | ABCA8 | 7,02 | 6,02 | 2 |
| ANKRD52 | 5,51 | 7,08 | -2,97 | GLRA2 | 4,34 | 3,34 | 2,01 |
| COBLL1 | 4,35 | 5,91 | -2,96 | PRND | 5,41 | 4,39 | 2,02 |
| CD53 | 3,61 | 5,14 | -2,89 | IBA57 | 7,02 | 6 | 2,02 |
| KIRREL3 | 3,96 | 5,45 | -2,81 | CABP1 | 6,01 | 4,98 | 2,04 |
| RAD54L | 5,37 | 6,86 | -2,81 | VPS37B | 7,9 | 6,87 | 2,04 |
| TCF4 | 4,06 | 5,54 | -2,79 | MLXIP | 6,7 | 5,67 | 2,04 |
| NDUFV3 | 3,97 | 5,43 | -2,75 | OR6C74 | 4,31 | 3,28 | 2,05 |
| HNRNPH1 | 6,58 | 8 | -2,67 | PRSS48 | 4,47 | 3,43 | 2,05 |
| RSPO2 | 3,84 | 5,25 | -2,66 | TMIGD2 | 6,55 | 5,51 | 2,06 |
| SNAPC4 | 5,53 | 6,93 | -2,64 | CLEC4G | 7,33 | 6,29 | 2,06 |
| KRTAP4-3 | 3,39 | 4,75 | -2,57 | SELL | 4,79 | 3,74 | 2,07 |
| NPL | 3,48 | 4,83 | -2,54 | PLEKHG6 | 4,34 | 3,29 | 2,07 |
| KRTAP1-5 | 6 | 7,34 | -2,53 | TBATA | 5,97 | 4,91 | 2,08 |
| MARCH1 | 3,46 | 4,77 | -2,47 | FAM209B | 4,98 | 3,93 | 2,08 |
| GPR32 | 4,5 | 5,78 | -2,44 | HBB | 5,99 | 4,94 | 2,08 |
| KRTAP22-2 | 3,95 | 5,23 | -2,42 | FDXR | 5,17 | 4,11 | 2,08 |
| PKP4 | 5,6 | 6,87 | -2,41 | MYOM2 | 5,59 | 4,52 | 2,09 |
| POGZ | 5,38 | 6,65 | -2,41 | CAPN12 | 5,5 | 4,44 | 2,09 |
| JAK2 | 4,88 | 6,13 | -2,38 | FNDC7 | 4,95 | 3,89 | 2,09 |
| TBC1D3H | 8,9 | 10,16 | -2,38 | PREX1 | 8,4 | 7,33 | 2,1 |
| C12orf80 | 3,89 | 5,12 | -2,35 | KCNN3 | 5,56 | 4,49 | 2,11 |
| FABP9 | 4,3 | 5,53 | -2,34 | MYO1G | 4,1 | 3,03 | 2,11 |
| FAM45A | 6,3 | 7,52 | -2,33 | GTF2A 1L | 5,24 | 4,17 | 2,11 |
| CNNM3 | 6,35 | 7,57 | -2,32 | FAM197Y1 | 5,08 | 3,99 | 2,12 |
| ZNF446 | 5,83 | 7,04 | -2,31 | HMHB1 | 8,03 | 6,94 | 2,13 |
| SGIP1 | 4,26 | 5,45 | -2,28 | ANKUB1 | 5,36 | 4,26 | 2,14 |
| LRRCC1 | 4,54 | 5,72 | -2,25 | DEFB105B/A | 4,9 | 3,79 | 2,15 |
| CAMKMT | 3,55 | 4,72 | -2,25 | OR11H2 | 4,55 | 3,44 | 2,15 |
| PLA2G2E | 5,67 | 6,83 | -2,23 | PTPRC | 5,89 | 4,79 | 2,15 |
| EFCAB5 | 4,75 | 5,91 | -2,23 | EXD3 | 8,66 | 7,55 | 2,17 |
| AKR1C8P | 3,84 | 4,99 | -2,22 | RLN1 | 4,51 | 3,39 | 2,17 |
| CUL9 | 4,07 | 5,22 | -2,22 | HKDC1 | 5,11 | 3,97 | 2,21 |
| MLXIP | 7,45 | 8,59 | -2,21 | ZNF790 | 7,84 | 6,7 | 2,21 |
| SPATA5 | 5,56 | 6,7 | -2,21 | DEC1 | 5,01 | 3,86 | 2,22 |
| IL12A | 5,87 | 7,01 | -2,2 | IGF1 | 6,59 | 5,44 | 2,22 |
| OR2Y1 | 3,33 | 4,47 | -2,2 | UNC119B | 5,11 | 3,94 | 2,25 |
| ZNF606 | 6,7 | 7,84 | -2,2 | ACKR3 | 6,91 | 5,72 | 2,28 |
| TBC1D3K | 7,23 | 8,36 | -2,19 | PDK4 | 4,72 | 3,53 | 2,28 |
| SH3GL3 | 4,24 | 5,36 | -2,16 | GRK4 | 6,09 | 4,88 | 2,3 |
| ID1 | 5,29 | 6,4 | -2,15 | METTL7B | 7,45 | 6,25 | 2, 3 |
| ITGAM | 3,87 | 4,97 | -2,14 | TMEM25 | 8,16 | 6,95 | 2,31 |
| PNPLA7 | 3,19 | 4,29 | -2,14 | SLC22A18AS | 7,81 | 6,59 | 2,33 |
| KRT23 | 4,56 | 5,66 | -2,14 | OR52E1 | 5,78 | 4,55 | 2,35 |
| HADHA | 4,6 | 5,69 | -2,13 | EFHC2 | 4,61 | 3,37 | 2,36 |
| TCERG1 | 4,02 | 5,11 | -2,13 | GAGE2D | 4,81 | 3,57 | 2,37 |
| GALNT14 | 4,75 | 5,84 | -2,13 | IL23R | 4,41 | 3,16 | 2,37 |
| DDX11 | 5,3 | 6,38 | -2,11 | RADIL | 4,6 | 3,36 | 2,38 |
| PPAP2B | 4,98 | 6,04 | -2,09 | THSD4 | 6,02 | 4,76 | 2,4 |
| ATP13A3 | 5,33 | 6,39 | -2,09 | FAM53B | 7,19 | 5,92 | 2,4 |
| PPM1L | 4,38 | 5,44 | -2,09 | TRIM31 | 5,27 | 4 | 2,42 |
| CCKBR | 6,32 | 7,38 | -2,08 | SEMA3A | 4,29 | 3,01 | 2,43 |
| PRELID3A | 5,97 | 7,03 | -2,08 | PLCB1 | 5,66 | 4,35 | 2,49 |
| TBC1D3G | 7,84 | 8,9 | -2,08 | GRIK2 | 7,07 | 5,76 | 2,49 |
| ZNF721 | 7,83 | 8,88 | -2,08 | PPP4R4 | 5,47 | 4,13 | 2,53 |
| ZNF536 | 4,31 | 5,36 | -2,08 | CCDC173 | 5,72 | 4,38 | 2,54 |
| MLXIP | 5,4 | 6,46 | -2,07 | CLYBL | 5,98 | 4,54 | 2,72 |
| CYP2R1 | 3,66 | 4,71 | -2,07 | SERINC5 | 5,59 | 4,09 | 2,84 |
| PTPN13 | 4,77 | 5,81 | -2,07 | OR2B6 | 5,85 | 4,34 | 2,85 |
| PRICKLE3 | 6,18 | 7,23 | -2,06 | AFF4 | 7,14 | 5,63 | 2,87 |
| DISP2 | 4,28 | 5,32 | -2,06 | PPM1B | 5,93 | 4,33 | 3,03 |
| XKR9 | 4,9 | 5,93 | -2,05 | LAP3 | 5,87 | 4,28 | 3,03 |
| SNX5 | 6,28 | 7,32 | -2,05 | OR9K2 | 8,03 | 6,19 | 3,57 |
| IL15 | 6,55 | 7,58 | -2,05 | | | | |
| OR8K5 | 3,55 | 4,58 | -2,04 | | | | |
| ATP2A3 | 8,33 | 9,36 | -2,04 | | | | |
| YWHAG | 5,33 | 6,35 | -2,03 | | | | |
| CCDC84 | 4,87 | 5,89 | -2,03 | | | | |
| TAS2R4 | 4,48 | 5,5 | -2,03 | | | | |
| TEF | 6,2 | 7,22 | -2,03 | | | | |
| OR5D13 | 3,81 | 4,83 | -2,02 | | | | |
| EFHB | 3,6 | 4,61 | -2,02 | | | | |
| ELF1 | 4,28 | 5,3 | -2,02 | | | | |
| NR2C2 | 5,61 | 6,62 | -2,02 | | | | |
| ITLN2 | 3,41 | 4,42 | -2,01 | | | | |
| JSRP1 | 3,38 | 4,39 | -2,01 | | | | |
| COL27A1 | 7,15 | 8,16 | -2,01 | | | | |
| TRIM27 | 4,16 | 5,16 | -2,01 | | | | |
| HSFX2 | 6,64 | 7,64 | -2 | | | | |
| MEDAG | 4,4 | 5,4 | -2 | | | | |

| **Table 7. Differentiation of gene expression of human corneal keratocytes after 24 hours of incubation with human C3a/C5a 0.1 µg/ml and human C3a/C5a 0.1 µg/ml with human C5L2 0.3 µg/ml.** | | | | | | | |
|---|---|---|---|---|---|---|---|
| **Gene Symbol** | **C3a/C5a Avg (log2)** | **C3a/C5a/C5L2 Avg (log2)** | **Fold Change** | **Gene Symbol** | **C3a/C5a Avg (log2)** | **C3a/C5a/C5L2 Avg (log2)** | **Fold Change** |
| ZNF512 | 4,99 | 6,44 | -2,73 | KBTBD8 | 6,84 | 5,83 | 2 |
| HYOU1 | 3,64 | 5,05 | -2,66 | SLC8A2 | 7,33 | 6,32 | 2,01 |
| DHX35 | 3,59 | 4,99 | -2,64 | STKLD1 | 6,46 | 5,45 | 2,01 |
| TAF1 | 4,2 | 5,57 | -2,58 | KRTAP5-8 | 5,4 | 4,39 | 2,01 |
| SRP72 | 4,69 | 6 | -2,47 | KPNA3 | 7,83 | 6,82 | 2,02 |
| MIR4738 | 3,78 | 5,07 | -2,45 | CFAP53 | 4,78 | 3,76 | 2,02 |
| | | | | CCDC85 | | | |
| SELL | 3,71 | 4,98 | -2,41 | A | 4,71 | 3,69 | 2,02 |
| SLC12A9 | 6,8 | 8,07 | -2,41 | CCDC33 | 9,42 | 8,4 | 2,03 |
| SPX | 4,33 | 5,56 | -2,35 | SCUBE1 | 7,27 | 6,24 | 2,04 |
| ATP8A2 | 3,94 | 5,14 | -2,29 | POTEM | 4,66 | 3,63 | 2,04 |
| | | | | LOC7999 | | | |
| SLC38A9 | 5,44 | 6,59 | -2,21 | 9 | 4,89 | 3,85 | 2,05 |
| FAM131 | | | | | | | |
| B | 5,14 | 6,27 | -2,19 | HIVEP2 | 4,27 | 3,22 | 2,06 |
| MARCH9 | 6,15 | 7,27 | -2,18 | MUC12 | 6,23 | 5,18 | 2,07 |
| LOC1053 | | | | | | | |
| 77348 | 4,56 | 5,68 | -2,18 | ESAM | 5,93 | 4,88 | 2,08 |
| ALKBH7 | 6,03 | 7,14 | -2,16 | DOCK3 | 5,79 | 4,74 | 2,08 |
| CPB1 | 3,11 | 4,22 | -2,16 | SYT12 | 5,42 | 4,36 | 2,08 |
| YEATS2 | 3,24 | 4,34 | -2,15 | DUS3L | 6,53 | 5,47 | 2,08 |
| MAGEE1 | 4,9 | 6 | -2,15 | OR5I1 | 4,71 | 3,65 | 2,09 |
| DFNB31 | 4,47 | 5,54 | -2,09 | IL7 | 5,07 | 4 | 2,1 |
| PSRC1 | 7,44 | 8,49 | -2,08 | GUCA1A | 5,92 | 4,85 | 2,1 |
| USP24 | 4,77 | 5,82 | -2,07 | C8orf46 | 6,35 | 5,27 | 2,11 |
| ACTR3C | 5,46 | 6,51 | -2,07 | GPR22 | 4,37 | 3,29 | 2,11 |
| PAK3 | 4,68 | 5,69 | -2,03 | CCDC110 | 4,71 | 3,63 | 2,12 |
| MPPE1 | 7,28 | 8,3 | -2,02 | OPCML | 4,66 | 3,57 | 2,12 |
| IKZF3 | 4,75 | 5,76 | -2,02 | PRKCH | 5,96 | 4,87 | 2,12 |
| KIRREL3 | 4,47 | 5,48 | -2,01 | THADA | 6,07 | 4,98 | 2,13 |
| MAK | 3,93 | 4,93 | -2,01 | PABPC4L | 7,6 | 6,5 | 2,13 |
| | | | | ARHGEF 1 | 6,38 | 5,29 | 2,14 |
| | | | | OR2B2 | 4,36 | 3,27 | 2,14 |
| | | | | FCAMR | 7,12 | 6,02 | 2,14 |
| | | | | CCDC38 | 5,81 | 4,69 | 2,17 |
| | | | | PHGR1 | 6,18 | 5,05 | 2,19 |
| | | | | SLC38A8 | 7,34 | 6,19 | 2,22 |
| | | | | LIPA | 5,74 | 4,58 | 2,23 |
| | | | | OR52E6 | 5,52 | 4,35 | 2,26 |
| | | | | DSG4 | 4,68 | 3,51 | 2,26 |
| | | | | LMOD3 | 4,87 | 3,69 | 2,26 |
| | | | | DDX11 | 6,55 | 5,37 | 2,27 |
| | | | | OR14A16 | 4,56 | 3,38 | 2,2 8 |
| | | | | C2orf83 | 6,42 | 5,22 | 2,29 |
| | | | | LAIR1 | 5,33 | 4,13 | 2,3 |
| | | | | TAL1 | 5,78 | 4,57 | 2,3 |
| | | | | CCSER1 | 4,47 | 3,25 | 2,32 |
| | | | | HMG20A | 6,67 | 5,45 | 2,33 |
| | | | | PTHLH | 6,57 | 5,35 | 2,33 |
| | | | | ACVRL1 | 7,01 | 5,78 | 2,34 |
| | | | | TEX29 | 5,9 | 4,68 | 2,34 |
| | | | | PADI4 | 5,84 | 4,58 | 2,39 |
| | | | | SH3TC1 | 8,17 | 6,91 | 2,39 |
| | | | | DHRSX | 5,26 | 3,99 | 2,42 |
| | | | | MOBP | 7,49 | 6,2 | 2,45 |
| | | | | PLGLB2 | 5,81 | 4,51 | 2,46 |
| | | | | P2RY 10 | 6 | 4,68 | 2,5 |
| | | | | TCAP | 9,99 | 8,52 | 2,76 |
| | | | | SRGN | 8,82 | 7,34 | 2,78 |

| **Table 8. Differentiation of gene expression of human corneal keratocytes after 24 hours of incubation with human C5L2 0.3 µg/ml and serum-free medium (control).** | | | | | | | |
|---|---|---|---|---|---|---|---|
| **Gene Symbol** | **C5L2 Avg (log2)** | **serumfree Avg (log2)** | **Fold Change** | **Gene Symbol** | **C5L2 Avg (log2)** | **serumfree Avg (log2)** | **Fold Change** |
| EPHA4 | 4,11 | 5,73 | -3,08 | AKR1C3 | 7,35 | 6,34 | 2 |
| PLEKHA6 | 4,46 | 6,03 | -2,97 | TTC28 | 6,57 | 5,57 | 2,01 |
| EPHA4 | 4,19 | 5,57 | -2,61 | AOC2 | 4,8 | 3,79 | 2,01 |
| SCAPER | 3,33 | 4,68 | -2,54 | FAM153A | 5,12 | 4,11 | 2,01 |
| CYP21A1P | 4,42 | 5,76 | -2,53 | PHACTR2 | 4,72 | 3,7 | 2,02 |
| SLC44A5 | 4,69 | 5,98 | -2,45 | ABCG2 | 6,25 | 5,23 | 2,02 |
| KDM1A | 3,76 | 5,03 | -2,41 | OR51B5 | 4,46 | 3,44 | 2,03 |
| SORBS2 | 3,1 | 4,37 | -2,41 | NCAM1 | 6,63 | 5,6 | 2,04 |
| MBOAT2 | 4,8 | 6,01 | -2,31 | OR4F6 | 4,24 | 3,21 | 2,05 |
| LINC00174 | 4,48 | 5,68 | -2,31 | OTOR | 5,88 | 4,85 | 2,05 |
| SKAP1 | 3,88 | 5,08 | -2,3 | EVPLL | 5,57 | 4,53 | 2,06 |
| CST9L | 3,83 | 5,01 | -2,26 | ZNF582-AS1 | 5,47 | 4,43 | 2,06 |
| SOHLH2 | 3,54 | 4,72 | -2,25 | RRP36 | 7,96 | 6,92 | 2,06 |
| ACSM1 | 3,11 | 4,28 | -2,25 | TMEM2 | 7,06 | 6,01 | 2,07 |
| PVRL4 | 6,58 | 7,73 | -2,23 | OSBPL1A | 4,23 | 3,18 | 2,07 |
| EFCAB10 | 5,59 | 6,74 | -2,23 | IKZF 1 | 4,33 | 3,27 | 2,08 |
| AF131215.3 | 4,86 | 6,02 | -2,23 | DACT1 | 5,63 | 4,57 | 2,08 |
| CLEC4A | 4,78 | 5,93 | -2,22 | SPATA19 | 4,53 | 3,47 | 2,08 |
| PLA2G5 | 5,54 | 6,67 | -2,2 | FLOT2 | 6,14 | 5,08 | 2,08 |
| PDK1 | 11,02 | 12,15 | -2,19 | EFCAB1 | 5,39 | 4,33 | 2,09 |
| CGNL1 | 3,89 | 5 | -2,16 | C8orf46 | 5,87 | 4,8 | 2,09 |
| MAGEA1 | 5,29 | 6,4 | -2,15 | SEMA5A | 7,18 | 6,11 | 2,1 |
| TRGJ1 | 3,47 | 4,57 | -2,14 | TSC1 | 7,5 | 6,43 | 2,1 |
| AGR2 | 3,74 | 4,84 | -2,14 | CCDC79 | 6,37 | 5,29 | 2,1 |
| USP49 | 6,45 | 7,54 | -2,13 | SLC22A5 | 7,42 | 6,34 | 2,11 |
| RPS3A | 5,91 | 7 | -2,13 | S100A7 | 4,1 | 3,02 | 2,11 |
| C20orf196 | 4,24 | 5,32 | -2,12 | LEAP2 | 5,06 | 3,98 | 2,12 |
| TJP3 | 2,99 | 4,06 | -2,11 | NSUN6 | 7,77 | 6,67 | 2,14 |
| WDR1 | 4,88 | 5,95 | -2,1 | MICALCL | 5,22 | 4,12 | 2,14 |
| CHST4 | 3,84 | 4,9 | -2,08 | RGS8 | 5,04 | 3,94 | 2,15 |
| MTM1 | 5,71 | 6,76 | -2,07 | YEATS2 | 5,58 | 4,47 | 2,15 |
| TRIM 10 | 5,55 | 6,6 | -2,07 | OR5AL1 | 4,58 | 3,47 | 2,17 |
| MYO1E | 5,47 | 6,52 | -2,07 | SPARCL1 | 5,14 | 4,01 | 2,19 |
| PDE1A | 3,16 | 4,21 | -2,07 | LONRF2 | 7,1 | 5,96 | 2,2 |
| FAM160A1 | 3,76 | 4,81 | -2,06 | TCF4 | 5,75 | 4,61 | 2,2 |
| ZBTB9 | 8,23 | 9,27 | -2,06 | NLGN4Y | 4,83 | 3,68 | 2,21 |
| OR5T1 | 4,35 | 5,38 | -2,05 | IFIH1 | 8,3 | 7,15 | 2,22 |
| PGK2 | 4,66 | 5,69 | -2,05 | DIO2 | 5,38 | 4,22 | 2,24 |
| C4orf50 | 4,93 | 5,96 | -2,04 | ATXN1 | 7,7 | 6,53 | 2,24 |
| NDUFA10 | 4,22 | 5,25 | -2,04 | MX1 | 7,68 | 6,51 | 2,25 |
| FEZF2 | 4,51 | 5,53 | -2,04 | FAM182B | 6,16 | 4,98 | 2,27 |
| EVI5 | 4,96 | 5,99 | -2,04 | ELF1 | 7,08 | 5,89 | 2,28 |
| SCIMP | 4,7 | 5,72 | -2,04 | TRIM27 | 5,89 | 4,69 | 2,29 |
| GBP4 | 4,75 | 5,77 | -2,03 | GHRHR | 5,62 | 4,41 | 2,32 |
| INPP5D | 3,66 | 4,68 | -2,03 | MS4A12 | 5,83 | 4,61 | 2,33 |
| NEK5 | 4,09 | 5,11 | -2,02 | VPS8 | 4,95 | 3,73 | 2,34 |
| BNIP3 | 13,97 | 14,97 | -2,01 | PATE1 | 5,97 | 4,74 | 2,34 |
| | | | | CDRT1 | 5,85 | 4,61 | 2,36 |
| | | | | HTN1 | 4,64 | 3,4 | 2,36 |
| | | | | KCNN3 | 5,48 | 4,24 | 2,37 |
| | | | | ZNF546 | 5,49 | 4,24 | 2,39 |
| | | | | MRAS | 6,63 | 5,31 | 2,5 |
| | | | | UBE2D3 | 5,33 | 3,99 | 2,53 |
| | | | | KIRREL3 | 5,43 | 4,05 | 2,61 |
| | | | | MYEF2 | 5,67 | 4,26 | 2,66 |
| | | | | IFI44L | 5,11 | 3,66 | 2,75 |
| | | | | SLC38A9 | 7,29 | 5,83 | 2,76 |
| | | | | LAP3 | 5,59 | 3,75 | 3,58 |

## Claims

1. Binder binding to complement-anaphylatoxin C5a and/or C3a and/or C4a and thereby preferably inhibiting the activity of C5a and/or C3a and/or C4a for use in the treatment of a subject having an ocular wound and/or fibrosis.

2. Binder for use in the treatment of a subject having an ocular wound and/or fibrosis according to claim 1 wherein said binder is selected from the group comprising a protein or a fragment thereof, a peptide, a non-IgG scaffold, an aptamer, oligonucleotides, an antibody or antibody-like proteins, peptidomimetics or a fragment thereof.

3. Binder for use in the treatment of a subject having an ocular wound and/or fibrosis according to claim 1 or 2 wherein said binder is a protein or a fragment thereof.

4. Binder according to any of claims 1 to 3 for use in the treatment of a subject having an ocular wound and/or fibrosis wherein said binder is administered to promote wound healing, in particular corneal wound healing.

5. Binder according to any of claims 1 to 4 for use in the treatment of a subject having an ocular wound and/or fibrosis wherein said binder binds to C5a and C3a and thereby essentially inhibiting the activity of C5a and C3a.

6. Binder according to any of claims 1 to 5 for use in the treatment of a subject having an ocular wound and/or fibrosis wherein said binder binds to C5a and C4a and thereby essentially inhibiting the activity of C5a and C4a.

7. Binder according to any of claims 1 to 6 for use in the treatment of a subject having an ocular wound and/or fibrosis wherein said binder binds to C3a and C4a and thereby essentially inhibiting the activity of C3a and C4a.

8. Binder according to any of claims 1 to 7 for use in the treatment of a subject having an ocular wound and/or fibrosis wherein said binder binds to C5a and C3a and C4a and thereby inhibiting the activity of C5a and C3a and C4a.

9. Binder according to any of claims 1 to 8 for use in the treatment of a subject having an ocular wound and/or fibrosis wherein said binder is a protein or protein fragment is selected from the group comprising human C5L2 protein according to SEQ ID No.: 1, a protein/peptide or fragment that is at least 60% identical to the full-length amino acid sequence of human C5L2 protein of SEQ ID No.:1, human C5aR1 protein according to SEQ ID No.: 2, a protein or fragment that is at least 60% identical to the full-length amino acid sequence of human C5aR1 protein of SEQ ID No.: 2, human C3aR protein according to SEQ ID No.: 3, a protein or fragment that is at least 60% identical to the full-length amino acid sequence of human C3aR protein as of SEQ ID No.: 3, a mouse C5L2 protein according to SEQ ID No.: 4, a protein or fragment that is at least 60% identical to the full-length amino acid sequence of mouse C5L2 protein of SEQ ID No.:4, mouse C5aR1 protein according to SEQ ID No.: 5, a protein or fragment that is at least 60% identical to the full-length amino acid sequence of mouse C5aR1 protein of SEQ ID No.: 5, mouse C3aR protein according to SEQ ID No.: 6, and a protein or fragment that is at least 60% identical to the full-length amino acid sequence of mouse C3aR protein of SEQ ID No.: 6.

10. Binder according to any of claims 1 to 9 for use in the treatment of a subject having an ocular wound and/or fibrosis wherein said binder is a protein/peptide or protein fragment and comprises at least one conserved region selected from the group comprising an amino acid sequence according to SEQ ID No.:7, an amino acid sequence according to SEQ ID No.:8, an amino acid sequence according to SEQ ID No.:9, an amino acid sequence according to SEQ ID No.:10, an amino acid sequence according to SEQ ID No.:11, an amino acid sequence according to SEQ ID No.:12, an amino acid sequence according to SEQ ID No.:13, an amino acid sequence according to SEQ ID No.:14, an amino acid sequence according to SEQ ID No.:15, an amino acid sequence according to SEQ ID No.:16, an amino acid sequence according to SEQ ID No.:17, and a protein or fragment that is at least 60% identical to any of the amino acid sequences according to SEQ ID No's.:7-17.

11. Binder according to claim 10 for use in the treatment of a subject having an ocular wound and/or fibrosis wherein said binder is a protein/peptide or protein fragment and comprises at least two conserved region selected from the group comprising an amino acid sequence according to SEQ ID No.:7, an amino acid sequence according to SEQ ID No.:8, an amino acid sequence according to SEQ ID No.:9, an amino acid sequence according to SEQ ID No.:10, an amino acid sequence according to SEQ ID No.:11, an amino acid sequence according to SEQ ID No.:12, an amino acid sequence according to SEQ ID No.:13, an amino acid sequence according to SEQ ID No.:14, an amino acid sequence according to SEQ ID No.:15, an amino acid sequence according to SEQ ID No.:16, an amino acid sequence according to SEQ ID No.:17, and a protein or fragment that is at least 60% identical to any of the amino acid sequences according to SEQ ID No's.:7-17.

12. Composition comprising at least two binders, preferably proteins or protein fragments, according to any of claims 1 to 11 for use in the treatment of a subject having an ocular wound and/or fibrosis.

13. Composition comprising at least three proteins or protein fragments according to any of claims 1 to 9 for use in the treatment of a subject having an ocular wound and/or fibrosis.

14. Pharmaceutical composition comprising a binder according to any of claims 1-11 or a composition according to claims 12 or 13 for use in the treatment of a subject having an ocular wound and/or fibrosis.

15. Binder according to any of claims 1-11 or a composition according to claims 12 or 13 or a pharmaceutical composition of claim 14 for use in the treatment of a subject wherein said subject suffers from a disease selected from the group comprising: conjunctivitis and conjunctival scars (including ocular pemphigoid), scleritis and episcleritis, corneal scars and opacities due to corneal ulcer, keratoconjunctivitis, keratitis, bullous keratopathy, corneal degenerations, iridocyclitis and adhesions of iris and ciliary body, chorioretinal scars/fibrosis due to chorioretinal inflammation or degeneration or haemorrhage or rupture or neovascularization, fibrotic vitreoretinopathies, such as in proliferative vitreoretinopathy, retinopathy of prematurity and diabetic retinopathy; choroidal neovascularization and degenerations of the macula, secondary glaucoma, endophthalmitis, and impairments of wound healing and fibrosis after ocular surgery or trauma, including intraocular foreign bodies.

16. Binder according to any of claims 1-11 or a composition according to claims 12 or 13 or a pharmaceutical composition of claims 14 for use in the treatment of a subject wherein said subject suffers from a disease selected from the group comprising: (idiopathic) pulmonary fibrosis, dermal keloid formation, sclerodermia, myelofibrosis, kidney-, pancreas- and heart-fibrosis, and fibrosis in (non)-alcoholic steatohepatosis, glomerulonephritis and (ANCA-associated) vasculitis.
